(19) Europäisches Patentamt
European Patent Office
Office européen des brevets

(11) Veröffentlichungsnummer: **0 177 443**
**B1**

(12) # EUROPÄISCHE PATENTSCHRIFT

(45) Veröffentlichungstag der Patentschrift: **18.10.89**

(51) Int. Cl.⁴: **C 07 F 9/38**, A 61 K 31/66

(21) Anmeldenummer: **85810352.6**

(22) Anmeldetag: **30.07.85**

(54) **Kristallmodifikation des Dinatrium-3-amino-1-hydroxy-propan-1,1-diphosphonates.**

(30) Priorität: **06.08.84 CH 3768/84**

(43) Veröffentlichungstag der Anmeldung:
**09.04.86 Patentblatt 86/15**

(45) Bekanntmachung des Hinweises auf die Patenterteilung:
**18.10.89 Patentblatt 89/42**

(84) Benannte Vertragsstaaten:
**AT BE CH DE FR GB IT LI LU NL SE**

(56) Entgegenhaltungen:
**DE-A-2 405 254**
**DE-A-2 553 963**
**US-A-4 304 734**

(73) Patentinhaber: **CIBA-GEIGY AG, Klybeckstrasse 141, CH-4002 Basel (CH)**
Patentinhaber: **Henkel Kommanditgesellschaft auf Aktien, Postfach 1100 Henkelstrasse 67, D-4000 Düsseldorf-Holthausen (DE)**

(72) Erfinder: **Stahl, Peter Heinrich, Dr., Lerchenstrasse 28, D-7800 Freiburg i/Br. (DE)**
Erfinder: **Schmitz, Beat, Dr., Maiengasse 27, CH-4123 Allschwil (CH)**

**EP 0 177 443 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine neue, kristallwasserhaltige, nichthygroskopische Kristallmodifikation des Dinatrium-3-amino-1-hydroxy-propan-1,1-diphosphonates der Formel

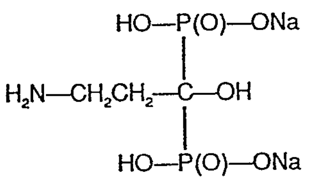

(I)

bzw. ein Verfahren zur Herstellung derselben.

Die dem Dinatriumsalz der Formel I zugrundeliegende 3-Amino-1-hydroxy-propan-1,1-diphosphonsäure, ein Verfahren zu und ihrer Herstellung ihre Verwendung als komplexbildender Waschmittelbestandteil ist in der DE-Auslegeschrift Nr. 2 130 794 beschrieben. Die genannte Säure sowie ihre wasserlöslichen Salze sind gemäss der DE-Offenlegungsschrift Nr. 2 405 254 auch als Arzneimittelwirkstoffe zur Behandlung von Störungen des Calcium- und Phosphatstoffwechsels und damit verbundener Erkrankungen von Warmblütern geeignet. Insbesondere kann gemäss der DE-Offenlegungsschrift Nr. 2 553 963 durch gleichzeitige orale Verabreichung von 3-Amino-1-hydroxy-propan-1,1-diphosphonsäure bzw. eines wasserlöslichen Salzes derselben, namentlich Dinatrium-3-amino-1-hydroxy-propan-1,1-diphosphonat (I), die für einen bestimmten therapeutischen Effekt erforderliche Calcitonindosis gegenüber einer Calcitonin-Monotherapie gesenkt werden.

Obgleich das Dinatrium-3-amino-1-hydroxy-propan-1,1-diphosphonat (I) sowie seine Verwendbarkeit als Arzneimittelwirkstoff somit als im Stande der Technik bekannt anzusehen ist, enthalten die genannten Druckschriften keine genauen Angaben zu seiner Herstellung. So ist der DE-Offenlegungsschrift Nr. 2 553 963 lediglich zu entnehmen, dass die 3-Amino-1-hydroxy-propan-1,1-diphosphonsäure «durch vollständige oder partielle Neutralisation in die gewünschten Salze übergeführt werden» kann.

Nach üblichem Neutralisationsverfahren erhält man das Dinatrium-3-amino-1-hydroxy-propan-1,1-diphosphonat jedoch in hygroskopischer Form und unbefriedigender Kristallinität. Führt man die Neutralisation beispielsweise in Anlehnung an die in Beispiel 2 der US-Patentschrift Nr. 4 304 734 beschriebenen Herstellungsweise des homologen Dinatrium-6-amino-1-hydroxy-hexan-1,1-diphosphonates durch (Vergleichsbeispiel 1), wird ein amorphes Produkt erhalten, das auch nach Trocknen bei etwa 60 °C unter vermindertem Druck bis zur Gewichtskonstanz noch hygroskopische Eigenschaften aufweist, d.h. in Abhängigkeit von Umgebungsfeuchtigkeit wechselnde Wassermengen aufnimmt. Dadurch wird seine Verarbeitung zu einer für die enterale, wie orale, Verabreichung geeigneten pharmazeutischen Darreichungsform sehr erschwert und die Lagerstabilität solcher Darreichungsformen in unvertretbarem Masse beeinträchtigt.

Versuche, durch Abwandlung der Neutralisations- oder Aufarbeitungsbedingungen eine definierte, bei annähernd normalen Umgebungsbedingungen lagerstabile kristalline Erscheinungsform des Dinatrium-3-amino-1-hydroxy-propan-1,1-diphosphonates zu erhalten und so die aufgezeigten Schwierigkeiten zu umgehen, führten zunächst nicht zum Erfolg. Je nach Verfahrensweise wurde die Bildung mehrerer verschiedener, anhand ihrer Röntgenpulverdiagramme und ihrer IR-Spektren unterscheidbarer fester Erscheinungsformen festgestellt. Engt man beispielsweise eine wässrige Lösung von Dinatrium-3-amino-1-hydroxy-propan-1,1-diphosphonat bei etwa 75 °C stark ein, lässt das Produkt durch langsames Abkühlen im Temperaturbereich von 45 °C bis 0 °C auskristallisieren, nutscht ab und trocknet bei Raumtemperatur unter vermindertem Druck bis zur Gewichtskonstanz (Beispiel 8), erhält man das Dinatrium-3-amino-1-hydroxy-propan-1,1-diphosphonat in einer nach den Charakteristiken seines Röntgenpulverdiagramms als «Modifikation B» bezeichneten Kristallform. Verfährt man analog, nimmt die Trocknung jedoch bei etwa 120 °C vor (Vergleichsbeispiel 2), erhält man eine weitere, nunmehr weitgehend wasserfreie, als «Modifikation A» bezeichnete Kristallform. Verfährt man wiederum analog, gibt jedoch nach dem Einengen bei 70–80 °C Äthanol hinzu, lässt unter Abkühlen kristallisieren und trocknet bei etwa 120 °C unter vermindertem Druck bis zur Gewichtskonstanz (Beispiel 5), erhält man die nur mässig kristalline «Modifikation C». Keine dieser Modifikationen wies die für einen enteralen, wie oralen, Arzneimittelwirkstoff zu fordernde Lagerstabilität auf. Hinzukommt, dass sich keine der erwähnten Verfahrensweisen reproduzierbar in wenigstens halbtechnischen Massstab übertragen liess.

Es bedurfte erst der überraschenden Feststellung, dass das Dinatrium-3-amino-1-hydroxy-propan-1,1-diphosphonat in einer nichthygroskopischen und damit lagerstabilen kristallwasserhaltigen, nachstehend als «Modifikation E» bezeichneten Kristallmodifikation erhalten wird, wenn man die Kristallbildung bei mindestens 50 °C einleitet und die Trocknung bei normaler oder leicht erhöhter Temperatur vornimmt, oder, wenn man wasserärmere feste Erscheinungsformen des Dinatrium-3-amino-1-hydroxy-propan-1,1-diphosphonates mit Wasser behandelt.

Die neue kristallwasserhaltige Kristallmodifikation des Dinatrium-3-amino-1-hydroxy-propan-1,1-diphosphonates (Modifikation E) weist ein charakteristisches Röntgenpulverdiagramm auf, welches von denen der Modifikationen A, B und C eindeutig unterschieden werden und zur Charakterisierung der neuen Modifikation E dienen kann. Zur Charakterisierung kann aber auch ihre Herstellungsweise herangezogen werden.

Die Modifikation E des Dinatrium-3-amino-1-hydroxy-propan-1,1-diphosphonates enthält 24,1 bis 24,5 Gew.-%, d.h. pro Mol desselben etwa 5

Mol Wasser. Es wird deshalb angenommen, dass es sich kristallchemisch um ein Pentahydrat handelt.

Dieses weist ausgezeichnete Kristallinität auf und ist bei annähernd normalen Umgebungstemperaturen völlig lagerstabil. So wurde festgestellt, dass weder Veränderung der relativen Luftfeuchtigkeit im Bereich von 10 bis 95%, noch Erwärmen auf etwas 60°C, selbst unter quasi-isothermen Bedingungen, noch 3-monatiges Lagern bei 40 bis 60°C 30 bis 55% relativer Luftfeuchtigkeit, noch 3-wöchiges Lagern bei Raumtemperatur und etwa 92% relativer Luftfeuchtigkeit eine nachweisbare Veränderung des Röntgenpulverdiagramms oder des IR-Spektrum bewirkt.

Die erfindungsgemässe Modifikation E des Dinatrium-3-amino-1-hydroxy-propan-1,1-diphosphonates kann dementsprechend ohne Schwierigkeiten zu lagerstabilen enteral, wie oral, applizierbaren Arzneimittelzubereitungen verarbeitet werden. Durch ihre Bereitstellung wird somit erstmalig ein praktikabler Weg zur Bereitstellung für die enterale, wie orale, Verabreichung bestimmter Arzneimittelzubereitungen des Dinatrium-3-amino-1-hydroxy-propan-1,1-diphosphonates eröffnet.

Die Erfindung betrifft dementsprechend ebenso ein neuartiges Verfahren zur Herstellung der neuen, nicht-hygroskopischen kristallwasserhaltigen Kristallmodifikation des Dinatrium-3-amino-1-hydroxypropan-1,1-diphosphonates. Dieses ist dadurch gekennzeichnet, dass man Dinatrium-3-amino-1-hydroxy-propan-1,1-diphosphonat aus wasserhaltiger Lösung zur Kristallisation bringt bzw. eine wasserärmere feste Erscheinungsform des Dinatrium-3-amino-1-hydroxy-propan-1,1-diphosphonates mit Wasser behandelt, jeweils das Produkt isoliert und bei normaler oder leicht erhöhter Temperatur trocknet.

Unter wasserhaltiger Lösung des Dinatrium-3-amino-1-hydroxy-propan-1,1-diphosphonates ist beispielsweise eine Lösung von Dinatrium-3-amino-1-hydroxy-propan-1,1-diphosphonat in Wasser oder einem Gemisch von Wasser und eines mit Wasser mischbaren organischen Lösungs- oder Verdünnungsmittels zu verstehen. Als Lösungs- oder Verdünnungsmittel kommen insbesondere niedere Akanole, d.h. Alkanole mit 1 bis 7, vor allem 1 bis 4 Kohlenstoffatomen, namentlich Äthanol oder in zweiter Linie Methanol, in Betracht. Bevorzugt sind wässrige oder wässrig-äthanolische Lösungen mit einem Äthanolgehalt von nicht mehr als 40 Vol.-%.

Wasserärmere feste Erscheinungsformen des Dinatrium-3-amino-1-hydroxy-propan-1,1-diphosphonates sind beispielsweise kristalline feste Erscheinungsformen desselben mit einem Kristallwassergehalt von weniger als etwa 24 Gew.-%, beispielsweise von 5 bis z.B. von 7,3 Gew.-%, insbesondere weitgehend wasserfreies Dinatrium-3-amino-1-hydroxy-propan-1,1-diphosphonat (Modifikation A) sowie die kristallwasserhaltige Modifikation C desselben.

Die Kristallisation des Dinatrium-3-amino-1-hydroxy-propan-1,1-diphosphonates aus wasserhaltiger Lösung erfolgt in üblicher Weise aus mindestens gesättigter Lösung, wobei zwischen einer Kristallbildungsphase und einer Kristallwachstumsphase unterschieden werden kann.

Die Kristallbildung kann spontan erfolgen, beispielsweise an in der Lösung enthaltenen festen Schwebeteilchen oder an der Oberfläche des Reaktionsgefässes bzw. Rührgerätes, wird aber zweckmässig durch Animpfen, d.h. Einbringen von Impfkristallen, ausgelöst. Stehen keine Impfkristalle zur Verfügung, können diese, vorteilhaft in einem aliquoten Teil der Lösung, in üblicher Weise, beispielsweise durch heftiges Schütteln, Einbringen von Glasstaub, Ritzen der Gefässwand oder Unterkühlen, hergestellt werden. Man kann aber auch das gesamte Lösungsvolumen geringfügig, d.h. um einige, z.B. 2 bis 5°C, unterkühlen, um die spontane Kristallbildung zu fördern, und dann wieder auf Ausgangstemperatur erwärmen.

Das Kristallwachstum wird insbesondere durch Erniedrigung der Sättigungskonzentration bewirkt, beispielsweise durch Abkühlen oder Hinzufügen eines Verdünnungsmittels, in welchem das Dinatrium-3-amino-1-hydroxy-propan-1,1-diphosphonat weniger löslich ist als in Wasser, wie eines der genannten mit Wasser mischbaren Verdünnungsmittel, oder durch Kombination beider und gegebenenfalls weiterer geeigneter Massnahmen. Dabei soll die Abkühlgeschwindigkeit bzw. die Zulaufgeschwindigkeit des Verdünnungsmittels so auf die Kristallwachsgeschwindigkeit abgestimmt werden, dass deutliche Übersättigung der Lösung vermieden wird.

Die gebildete neue, kristallwasserhaltige Kristallmodifikation kann mit Hilfe einer beliebigen für die Trennung binärer fest/flüssiger Systeme zur Verfügung stehenden Methode aus ihrer wasserhaltigen Suspension isoliert werden, beispielsweise durch Filtration, Druckfiltration («Abnutschen»), Zentrifugieren oder Dekantieren. Zur Entfernung von in verbleibenden Mutterlaugeresten enthaltenen Verunreinigungen kann mit Wasser oder vorzugsweise einem wässrigen Alkanol mit 1 bis 7, vor allem 1 bis 4 Kohlenstoffatomen, z.B., mit ungefähr 40 bis 60%igem Äthanol oder 50 bis 75%igem Methanol, nachgewaschen werden.

Die Trocknung bei normaler oder leicht erhöhter Temperatur wird beispielsweise im Temperaturbereich von 15 bis 60°C, vorzugsweise bei 18 bis 25°C (Raumtemperatur) oder bei 35 bis 40°C, durchgeführt und bis zu annähernder Gewichtskonstanz fortgesetzt. Zur Beschleunigung der Trocknung kann unter vermindertem Druck gearbeitet werden, wobei sogenanntes Wasserstrahlvakuum (5 bis 25 mbar) durchaus genügt.

Die Herstellung mindestens gesättigter Lösungen des Dinatrium-3-amino-1-hydroxy-propan-1,1-diphosphonates erfolgt in üblicher Weise, beispielsweise durch Auflösen einer festen Erscheinungsform derselben oder vorteilhaft in situ durch partielle Neutralisation von 3-Amino-1-hydroxy-propan-1,1-diphosphonsäure mit einem

basischen Natriumsalz in wasserhaltigem Milieu und erforderlichenfalls Überführung einer zunächst ungesättigten Lösung des Dinatrium-3-amino-1-hydroxy-propan-1,1-diphosphonates in eine gesättigte.

Für die partielle Neutralisation der 3-Amino-1-hydroxy-propan-1,1-diphosphonsäure geeignete basische Natriumsalze sind beispielsweise Natriumhydroxid oder Natriumcarbonat.

Vorzugsweise setzt man 3-Amino-1-hydroxy-propan-1,1-diphosphonsäure in wässriger Suspension mit mindestens der für die Bildung des Dinatrium-3-amino-1-hydroxy-propan-1,1-diphosphonates erforderlichen Menge wässriger Natronlauge um, d.h. man fügt zu einer wässrigen Suspension von 3-Amiono-1-hydroxy-propan-1,1-diphosphonsäure soviel wässrige, vorzugsweise 30 bis 40%ige, Natronlauge hinzu, bis der pH-Wert der Reaktionsmischung mindestens 7, vorzugweise 7,2 bis 7,5 beträgt.

Die Überführung einer ungesättigten, vorzugsweise wässrigen Lösung von Dinatrium-3-amino-1-hydroxy-propan-1,1-diphosphonat in eine gesättigte erfolgt in üblicher Weise, beispielsweise durch Einengen, d.h. Verdampfen überschüssigen Lösungsmittels, vorzugsweise von Wasser, Hinzufügen eines mit Wasser mischbaren Verdünnungsmittels, in welchem Dinatrium-3-amino-1-hydroxy-propan-1,1-diphosphonat weniger löslich ist als im Wasser oder, indirekt, durch Erniedrigung der Sättigungskonzentration von Dinatrium-3-amino-1-hydroxy-propan-1,1-diphosphonat, z.B. durch Abkühlen oder, in zweiter Linie, gleichionigen Zusatz, wie Aussalzen.

Die Sättigungskonzentration des Dinatrium-3-amino-1-hydroxy-propan-1,1-diphosphonates in wasserhaltiger Lösung ist stark temperaturabhängig. Das gestattet, durch geeignete Wahl der Ausgangstemperatur die Kristallisation so zu lenken, dass das Kristallwachstum überwiegend im bequem beherrschbaren Temperaturbereich von 0 bis 50°C erfolgt. Man leitet die Kristallbildung dementsprechend bei mindestens 50°C, vorzugsweise bei 50 bis 80°C, insbesondere 55 bis 70°C, ein. Dadurch wird sichergestellt, dass bei 0 bis 5°C mindestens 95% des gelösten Dinatrium-3-amino-1-hydroxy-propan-1,1-diphosphonates in Form der erfindungsgemässen kristallwasserhaltigen Kristallmodifikation (Modifikation E) auskristallisieren. Wird die Kristallbildung unterhalb etwa 50°C, z.B. bei 45°C, eingeleitet, kann sich die Modifikation B bilden, die sich jedoch durch Erhitzen in die Modifikation A umwandeln lässt, die ihrerseits durch Behandlung mit Wasser in die erfindungsgemässe Modifikation E überführt werden kann.

In einer bevorzugten Ausführungsform des vorstehend beschriebenen Kristallisationsverfahrens engt man beispielsweise eine 10- bis 55%ige, vorzugsweise 12- bis 28%ige, wässrige Lösung von Dinatrium-3-amino-1-hydroxy-propan-1,1-diphosphonat bei 70 bis 80°C, z.B. bei etwa 75°C, bis zur Sättigungskonzentration des Dinatrium-3-amino-1-hydroxy-propan-1,1-diphosphonates ein, leitet die Kristallbildung ein, kühlt langsam auf 0 bis 5°C ab und trocknet das isolierte Produkt bei 20 bis 40°C, vorzugsweise bei 35 bis 40° bis zur annähernden Gewichtskonstanz.

In einer anderen, besonders bevorzugten Ausführungsform kühlt man eine 35- bis 45%ige, z.B. etwa 40%ige, auf 70 bis 80°C erwärmte wässrige Lösung langsam auf nicht weniger als 55°C ab, bis die Kristallbildung einsetzt, kühlt dann langsam, z.B. innerhalb von etwa 2 Stunden, zunächst auf 20 bis 25°C ab, fügt 35 bis 45, z.B. etwa 40 Vol-%, (bezogen auf die Gesamtwassermenge) Äthanol hinzu, kühlt langsam, z.B. innerhalb einer Stunde, weiter auf 0 bis 5°C, lässt einige Zeit, z.B. etwa 1 Stunde, bei dieser Temperatur rühren und trocknet das isolierte Produkt bei 20 bis 40°C, vorzugsweise bei 35 bis 40°C, bis zur annähernden Gewichtskonstanz.

Die bei der Neutralisation von 3-Amino-1-hydroxy-propan-1,1-diphosphonsäure freiwerdende Neutralisationswärme kann vorteilhaft genutzt werden, um zumindest einen Teil des Energiebedarfes für die Erreichung der Kristallbildungstemperatur zu decken.

Die Erfindung betrifft somit ebenso ein Verfahren zur Herstellung des kristallinen, kristallwasserhaltigen Dinatrium-3-amino-1-hydroxy-propan-1,1-diphosphonates in nicht-hygroskopischer Form durch Umsetzung von 3-Amino-1-hydroxy-propan-1,1-diphosphonsäure mit einem für deren partielle Neutralisation geeigneten basischen Natriumsalz, durch das die neue, nichthygroskopische Modifikation E erzielt wird. Dieses ist dadurch gekennzeichnet, dass man 3-Amino-1-hydroxy-propan-1,1-diphosphonsäure in wasserhaltiger Suspension und/oder Lösung mit der erforderlichen Menge wässriger Natronlauge umsetzt, das gebildete Dinatrium-3-amino-1-hydroxy-propan-1,1-diphosphonat aus mindestens gesättigter wasserhaltiger Lösung in Form der neuen, kristallwasserhaltigen Kristallform zur Kristallisation bringt, wobei man die Kristallbildung bei mindestens 50°C einleitet, das Produkt isoliert und bei normaler oder leicht erhöhter Temperatur trocknet.

Die Reaktionsbedingungen werden dabei vorteilhaft so gewählt, dass die Neutralisation im Temperaturbereich von 35 bis 85°C, vorzugsweise von 35 bis 55°C oder von 50 bis 85°C, die Kristallbildung bei mindestens etwa 50°C, beispielsweise bei 50 bis 75°C, vorzugsweise bei 50 bis 55°C, oder bei 55 bis 70°C, und das Kristallwachstum überwiegend im Temperaturbereich von 50 bis 0°C erfolgt.

In einer bevorzugten Ausführungsform dieser die Kristallisation des Dinatrium-3-amino-1-hydroxy-propan-1,1-diphosphonates in der neuen, kristallwasserhaltigen Kristallmodifikation aus mindestens gesättigter wasserhaltiger Lösung und die in situ-Herstellung einer solchen Lösung kombinierenden Verfahrensweise neutralisiert man beispielsweise 10 Gewichtsteile 3-Amino-1-hydroxy-propan-1,1-diphosphonsäure, suspendiert in 18 bis 23, z.B. etwa 20, Volumenteilen Wasser bei 65 bis 85°C, mit der für die Bildung

des Dinatrium-3-amino-1-hydroxy-propan-1,1-diphosphonates erforderlichen Menge 27,5 bis 32,5%iger, z. B. 8,5 bis 8,7, d. h. etwa 8,6 Volumenteilen (etwa 11,4 Gewichtsteilen), ungefähr 30%iger, wässriger Natronlauge, filtriert erforderlichenfalls die gebildete Lösung, wäscht das Filtrat mit 1,2 bis 1,6, z.B. etwa 1,4,Volumenteilen Wasser nach, lässt bis mindestens zur Sättigung, beispielsweise auf 70 bis 55°C, abkühlen, impft an, kühlt langsam, beispielsweise innerhalb von etwa 2 Stunden, auf 20 bis 25°C, fügt 7 bis 12, z.B. etwa 10, Volumenteile Äthanol hinzu, kühlt langsam, beispielsweise innerhalb einer Stunde, weiter auf 0 bis 5°C, lässt bei dieser Temperatur einige Zeit, beispielsweise 1 bis 15 Stunden, nachrühren, nutscht oder zentrifugiert ab, wäscht in Portionen mit insgesamt 7,5 bis 12,5, z.B. etwa 10, Volumenteilen 40 bis 60%igen, z.B. 50%igen, wässrigen Äthanols nach und trocknet bei leicht erhöhter Temperatur, beispielsweise 30 bis 60°C, vorzugsweise bei 35 bis 40°C, vorteilhaft unter vermindertem Druck, bis zur Gewichtskonstanz.

In einer anderen bevorzugten Ausführungsform suspendiert man beispielsweise 10 Gewichtsteile 3-Amino-1-hydroxy-propan-1,1-diphosphonsäure, in 25 bis 35, z.B. etwa 32,5, Volumenteilen Wasser, erwärmt auf 35°C, gibt dann die für die Bildung des Dinatrium-3-amino-1-hydroxy-propan-1,1-diphosphonates erforderlichen Menge 27,5 bis 32%iger, z. B. 8,5 bis 8,7, d. h. etwa 8,6 Volumenteile (etwa 11,4 Gewichtsteile, ungefähr 30%iger, wässriger Natronlauge so schnell hinzu, dass die Temperatur auf 52 bis 58°C, z.B. auf etwa 54°C, steigt, leitet ungefähr bei dieser Temperatur die Kristallisation ein, lässt unter Rühren langsam auf Raumtemperatur abkühlen, saugt ab, wäscht portionsweise mit insgesamt 15 bis 25, z.B. etwa 18 Volumenteilen, 50 bis 75%igen, z.B. ungefähr 66%igen, wässrigen Methanols nach und trocknet wie vorstehend angegeben.

Die Behandlung im Vergleich mit der erfindungsgemässen Modifikation E wasserärmerer fester Erscheinungsformen des Dinatrium-3-amino-1-hydroxy-propan-1,1-diphosphonates mit Wasser erfolgt in üblicher Weise durch Einwirkung mindestens der für die Bildung der neuen, kristallwasserhaltigen Form erforderlichen Wassermenge. Das Wasser kann dabei in flüssigem oder gasförmigen Aggregatzustand vorliegen.

Bei der Behandlung mit Wasser in flüssigem Zustand ist die anwendbare Wassermenge nach oben durch die Löslichkeit des Dinatrium-3-amino-1-hydroxy-propan-1,1-diphosphonates begrenzt; sie darf den zur vollständigen Auflösung erforderlichen Grenzwert, d.h. das durch den um 1 verminderten Kehrwert der Sättigungskonzentration des Dinatrium-3-amino-1-hydroxy-propan-1,1-diphosphonates gegebene Vielfache nicht erreichen. Andererseits werden ausgehend von weitgehend wasserfreiem Dinatrium-3-amino-1-hydroxy-propan-1,1-diphosphonat mindestens etwa 32 Gew.-% Wasser benötigt. Als optimal hat sich die 2- bis 3-fache, z.B. 2,5-fache, Gewichtsmenge Wasser erwiesen. Die Temperatur, bei der die Behandlung mit Wasser im flüssigen Aggregatzustand durchgeführt wird, ist an sich nicht kritisch. Um Verluste durch die mit der Temperatur stark ansteigende Löslichkeit möglichst gering zu halten, empfiehlt es sich jedoch, bei normaler oder, insbesondere gegen Ende der Behandlung, leicht erniedrigter Temperatur, z.B. bei 15 bis 30°C, insbesondere 20 bis 25°C, und gegen Ende bei 0 bis 5°C, zu arbeiten.

Bei der Behandlung mit Wasser im dampfförmigen Aggregatzustand setzt man das Ausgangsmaterial einer wasserdampfhaltigen Atmosphäre, z.B. feuchter Luft, aus. Die für die Umwandlung erforderliche Expositionsdauer nimmt mit zunehmender relativer Luftfeuchtigkeit ab. Diese sollte deshalb etwa 90% nicht wesentlich unterschreiten und im Hinblick auf mögliche Kondensation von Wasser an der Kristalloberfläche etwas weniger als 100% betragen. Bei 20 bis 25°C hat sich feuchte Luft der relativen Luftfeuchtigkeit von 95 bis 99%, z.B. von etwa 97%, als gut geeignet erwiesen.

In einer bevorzugten Ausführungsform dieser Verfahrensvariante lässt man beispielsweise auf weitgehend wasserfreies Dinatrium-3-amino-1-hydroxy-propan-1,1-diphosphonat oder auf eine andere wasserärmere kristalline Form, z.B. die Modifikation C, desselben bei 20 bis 25°C feuchte Luft der relativen Luftfeuchtigkeit von 90 bis 99%, z.B. von 95 bis 99%, bis zur Aufnahme mindestens der erforderlichen Wassermenge, d.h. von mindestens etwa 32,2 Gew.-%, z.B. von 32,2 bis 37,5 Gew.-%, (unter Berücksichtigung des Ausgangswassergehalts) einwirken und senkt dann die Luftfeuchtigkeit wieder auf normale Umgebungswerte, d.h. auf 35 bis 80%, z.B. auf 40 bis 60%.

Die Herstellung als Ausgangsmaterial dienender wasserärmerer fester Erscheinungsformen des Dinatrium-3-amino-1-hydroxy-propan-1,1-diphosphonates erfolgt durch partielle Neutralisation von 3-Amino-1-hydroxy-propan-1,1-diphosphonsäure mit Natriumhydroxid oder Natriumcarbonat, Kristallisation des gebildeten Dinatrium-3-amino-1-hydroxy-propan-1,1-diphosphonates aus mindestens gesättigten wasserhaltigen Lösung und Isolierung desselben, wie vorstehend beschrieben, und geeignete Nachbehandlung. Diese besteht in einer scharfen Trocknung durch Erwärmen auf 100 bis 160°C beispielsweise auf 120 bis 150°C.

So erhält man weitgehend wasserfreies Dinatrium-3-amino-1-hydroxypropan-1,1-diphosphonat (Modifikation A) beispielsweise, indem man zu einer Suspension von 10 Gewichtsteilen 3-Amino-1-hydroxy-propan-1,1-diphosphonsäure in 25 bis 37,5, z.B. etwa 32 Volumenteilen Wasser die für die Bildung des Dinatrium-3-amino-1-hydroxy-propan-1,1-diphosphonates erforderlichen Menge 25- bis 45%iger, z.B. 8,4 bis 8,5, d.h.etwa 8,5 Gewichtsteile, ungefähr 40%iger, wässriger Natronlauge so schnell hinzugibt, dass die Reaktionstemperatur auf 50 bis 55°C steigt, bei 45 bis 52°C die Kristallbildung einleitet, langsam auf Raumtemperatur abkühlt, absaugt, in

Portionen mit insgesamt etwa 10 bis 20, z. B. etwa 16 Volumenteilen 50- bis 75%igen, z. B. ungefähr 66%igen, wässrigen Methanols wäscht und unter vermindertem Druck, bei 120 bis 150°C bis zur Gewichtskonstanz trocknet.

Zur Herstellung der als Ausgangsmaterial verwendbaren kristallinen Modifikation C des Dinatrium-3-amino-1-hydroxy-propan-1,1-diphosphonates erwärmt man beispielsweise eine Suspension von 10 Gewichtsteilen 3-Amino-1-hydroxy-propan-1,1-diphosphonsäure in 70 bis 100, z.B. etwa 92 Gewichtsteilen Wasser auf etwa 60°C, gibt die für die Bildung des Dinatrium-3-amino-1-hydroxy-propan-1,1-diphosphonates erforderliche Menge 27,5- bis 32,5%iger, z. B. 8,5 bis 8,7, d.h. etwa 8,6 Volumenteile (etwa 11,4 Gewichtsteile), ungefähr 30%iger, wässriger Natronlauge hinzu, dampft bei etwa 75°C etwa 50 Teile Wasser ab, bis die Kristallisation beginnt, fügt 10 bis 15, z. B. etwa 12,8 Volumenteile Äthanol hinzu, kühlt dann auf Raumtemperatur, rührt 1 Stunde bei 0 bis 5°C aus, saugt ab, wäscht mit etwa 20 Volumenteilen etwa 66%igem Äthanol nach und trocknet unter vermindertem Druck bei etwa 120°C bis zu annähernden Gewichtskonstanz.

Die neue kristallwasserhaltige Kristallmodifikation (Modifikation E) des Dinatrium-3-amino-1-hydroxy-propan-1,1-diphosphonates ist auf Grund ihrer vorzüglichen Lagerstabilität, wie bereits erwähnt, die als Arzneimittelwirkstoff in für die zur enteralen, wie oralen, aber auch rektalen, Verabreichung bestimmten Arzneimittelzubereitungen derzeit bestgeeignete feste Erscheinungsform des Dinatrium-3-amino-1-hydroxy-propan-1,1-diphosphonates.

Die Erfindung betrifft dementsprechend auch die Verwendung dieser neuen kristallwasserhaltigen Kristallmodifikation des Dinatrium-3-amino-1-hydroxy-propan-1,1-diphosphonates als Wirkstoff in bzw. zur Herstellung von für die enterale, vorzugsweise orale, Verabreichung bestimmten pharmazeutischen Präparaten, sowie für die enterale, vorzugsweise orale, Verabreichung bestimmte pharmazeutische Präparate, enthaltend die neue, kristallwasserhaltige Kristallmodifikation (Modifikation E) des Dinatrium-3-amino-1-hydroxy-propan-1,1-diphosphonates.

Bei den erfindungsgemässen pharmazeutischen Präparaten handelt es sich insbesondere um solche zur oralen, ferner rektalen Verabreichung an Warmblüter, welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Im Normalfall ist für einen etwa 75 kg schweren Warmblüter bei oraler Applikation eine ungefähre Tagesdosis von 0,2 bis 200, insbesondere 1 bis 50, mg/kg, vorteilhaft in mehreren gleichen Teildosen zu veranschlagen.

Die neuen pharmazeutischen Präparate enthalten z. B. von 10 bis 80%, vorzugsweise von 20 bis 60% des Wirkstoffs, erfindungsgemäss pharmazeutische Präparate zur enteralen Verabreichung sind z. B. solche in Dosiseinheitsformen wie Dragées, Tabletten, Kapseln oder Suppositorien. Diesen werden in an sich bekannter Weise, z. B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Überzügen versehen, wobei man u. a. konzentrierte Zuckerlösungen, welche gegebenenfalls arabischen Gummi, Talk, Polyvinylpyrrolidon, Polyäthylenglykol und/oder Tritandioxid enthalten, Lacklösungen in geeigneten organischen Lösungsmitteln oder Lösungsmittelgemischen oder, zur Herstellung von magensaftresistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z. B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z. B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen gelöst oder suspendiert, wobei ebenfalls Stabilisatoren zugefügt sein können.

Als rektal anwendbare pharmazeutische Präparate kommen Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z. B. flüssige Triglyceride, Polyäthylenglykole oder Paraffinkohlenwasserstoffe in Frage.

Die nachfolgenden Beispiele illustrieren die oben beschriebene Erfindung; sie sollen jedoch diese in ihrem Umfang in keiner Weise einschränken, Temperaturen sind in Celsiusgraden, Drucke in mbar angegeben.

Beispiel 1

74,2 g weitgehend wasserfreies Dinatrium-3-amino-1-hydroxy-propan-1,1-diphosphonat werden unter Rühren in einem auf 75° geheizten Wasserbad in 500 ml entmineralisiertem Wasser gelöst. Man engt unter vermindertem Druck langsam ein, bis Kristallisation einsetzt, was nach Abziehen von etwa 375 ml Wasser erfolgt, und lässt unter Rühren langsam auf Raumtemperatur abkühlen. Nach Stehenlassen über Nacht lässt man 1 Stunde im Eisbad rühren, saugt ab, wäscht mit wenig eiskaltem Wasser nach und trocknet unter etwa 20 mbar bei Raumtemperatur bis zur Gewichtskonstanz. Man erhält so das Dinatrium-3-amino-1-hydroxy-propan-1,1-diphosphonat in Form der neuen, kristallwasserhaltigen Kristallmodifikation (Modifikation E). Diese ist durch folgende Gitterabstände (d-Werte) und relativen Linienintensitäten (Intensitäten) ihres Röntgenpulverdiagramms (Kamera nach Guinier-de-Wolff, Strahlungsquelle: Kupfer-$K_\alpha$) charakterisiert:

| d-Werte (Ångström) | Intensität |
|---|---|
| 10.2 | mittel |
| 9.9 | mittel |
| 9.2 | sehr stark |
| 5.91 | sehr stark |
| 5.57 | stark |
| 5.42 | sehr schwach |
| 5.30 | stark |
| 5.14 | mittel |
| 5.02 | sehr stark |
| 4.97 | sehr stark |
| 4.63 | sehr schwach |
| 4.41 | stark |
| 4.16 | stark |
| 4.07 | schwach |
| 4.04 | mittel |
| 3.95 | sehr schwach |
| 3.75 | stark |
| 3.67 | schwach |
| 3.63 | mittel |
| 3.61 | sehr schwach |
| 3.58 | sehr schwach |
| 3.51 | mittel |
| 3.43 | stark |
| 3.38 | mittel |
| 3.15 | mittel |
| 3.14 | mittel |
| 3.09 | mittel |
| 3.03 | sehr stark |
| 3.01 | sehr stark |
| 2.98 | stark |
| 2.97 | sehr schwach |
| 2.91 | sehr stark |
| 2.82 | stark |
| 2.80 | mittel |
| 2.78 | mittel |
| 2.75 | stark |
| 2.73 | schwach |
| 2.71 | schwach |
| 2.69 | schwach |
| 2.67 | mittel |
| 2.66 | mittel |
| 2.63 | stark |
| 2.62 | stark |
| 2.61 | stark |
| 2.58 | sehr stark |
| 2.57 | sehr stark |

Das Ausgangsmaterial kann beispielsweise folgendermassen hergestellt werden:

77,1 g 3-Amino-1-hydroxy-propan-1,1-diphosphonsäure werden in 250 ml entmineralisiertem Wasser suspendiert und unter kräftigem Rühren mit 65,6 g 40%iger wässriger Natronlauge versetzt. Die Zulaufgeschwindigkeit derselben wird so einreguliert, dass die Temperatur der Reaktionsmischung während der Neutralisation auf etwa 50°C steigt. Man erwärmt auf 52°C, impft an, lässt langsam auf Raumtemperatur abkühlen, lässt über Nacht stehen, saugt den erhaltenen Salzkuchen ab, wäscht mit 120 ml etwa 66%igem Methanol und trocknet unter 10–20 mbar bei 150°C bis zur Gewichtskonstanz. Man erhält das Dinatrium-3-amino-1-hydroxy-propan-1,1-diphosphonat in kristalliner, weitgehend wasserfreien Form (Modifikation A). Diese ist durch folgende Gitterabstände (d-Werte) und relativen Linienintensitäten (Intensitäten) ihres Röntgenpulverdiagramms (Kamera nach Guinier-de-Wolff, Strahlungsquelle: Kupfer-$K_\alpha$) charakterisiert:

| d-Werte (Ångström) | Intensität |
|---|---|
| 11.0 | stark |
| 7.0 | sehr stark |
| 5.91 | mittel |
| 5.69 | sehr stark |
| 5.63 | sehr schwach |
| 5.52 | mittel |
| 5.21 | stark |
| 4.96 | schwach |
| 4.84 | sehr schwach |

| d-Werte (Ångström) | Intensität |
|---|---|
| 4.73 | mittel |
| 4.63 | schwach |
| 4.54 | sehr stark |
| 4.19 | mittel |
| 3.90 | stark |
| 3.83 | sehr schwach |
| 3.79 | sehr schwach |
| 3.74 | stark |
| 3.53 | stark |
| 3.47 | mittel |
| 3.44 | sehr stark |
| 3.35 | mittel |
| 3.32 | mittel |
| 3.18 | mittel |
| 3.14 | sehr schwach |
| 3.09 | sehr schwach |
| 2.98 | schwach |
| 2.94 | mittel |
| 2.90 | stark |
| 2.85 | stark |
| 2.77 | stark |
| 2.75 | sehr schwach |
| 2.72 | schwach |
| 2.68 | schwach |
| 2.67 | mittel |
| 2.63 | schwach |
| 2.61 | stark |
| 2.54 | stark |
| 2.48 | sehr stark |
| 2.46 | sehr stark |

Beispiel 2

40,0 g weitgehend wasserfreies Dinatrium-3-amino-1-hydroxy-propan-1,1-diphosphonat werden in 125 ml entmineralisiertem Wasser suspendiert und unter heftigem Rühren bis zur vollständigen Auflösung erwärmt. Die bei 59,5° gebildete klare Lösung wird zunächst auf 52° abgekühlt und nach Einsetzen der Kristallisation langsam auf Raumtemperatur weitergekühlt. Man lässt 8 Stunden bei Raumtemperatur weiterrühren, saugt ab, wäscht mit etwa 60 ml eines Gemisches aus 2 Volumenteilen Methanol und 1 Volumenteil Wasser und trocknet bei 28–31° unter etwa 20 mbar bis zur Gewichtskonstanz. Man erhält das Dinatrium-3-amino-1-hydroxy-propan-1,1-diphosphonat in der neuen, kristallwasserhaltigen Kristallform (Modifikation E), deren IR-Spektrum mit dem des gemäss Beispiel 1 erhaltenen Produktes identisch ist.

Beispiel 3

70,0 kg 3-Amino-1-hydroxy-propan-1,1-diphosphonsäure werden unter Rühren in 140 l entmineralisiertem Wasser suspendiert. Man erwärmt auf 65–70° und lässt 59,9 l (79,5 kg) 30%ige wässrige Natronlauge so schnell zulaufen, dass die Temperatur der Reaktionsmischung unter Bildung einer klaren Lösung auf etwa 85° steigt. Der pH-Wert der Lösung soll zwischen 7,2 und 7,5 liegen. Er kann erforderlichenfalls durch Zugabe kleiner Mengen 30%iger Natronlauge bzw. von 3-Amino-1-hydroxy-propan-1,1-diphosphonsäure feineingestellt werden. Die Lösung wird durch einen mit wenig Diatomeenerde belegten, auf 75–80° vorgeheizten Filter in ein auf die gleiche Temperatur vorgeheiztes Gefäss klarfiltriert. Man impft an, kühlt unter Rühren innerhalb von 2 Stunden auf 20–25°, lässt innerhalb weiterer 2 Stunden unter Rühren 70 l 96%iges Äthanol zulaufen, kühlt dann innerhalb 1 Stunde auf 0–5° und lässt mindestens 1 Stunde bei dieser Temperatur ausrühren. Dann wird von der Mutterlauge abzentrifugiert und portionsweise mit einem Gemisch aus 25 l Äthanol und 45 l entmineralisiertem Wasser gewaschen. Man lässt gut ausschwingen und trocknet im Vakuumschrank bei 35–40° bie zur Gewichtskonstanz (etwa 24 Stunden). Man erhält das Dinatrium-3-amino-1-hydroxy-propan-1,1-diphosphonat in der neuen, kristallwasserhaltigen Kristallform (Modifikation E), deren IR-Spektrum mit dem des gemäss Beispiel 1 erhaltenen Produktes identisch ist.

Beispiel 4

32,7 g 3-Amino-1-hydroxy-propan-1,1-diphosphonsäure werden in 106 ml entmineralisiertem Wasser suspendiert. Man erwärmt auf 35° und tropft unter lebhaftem Rühren 28 ml 30%ige wässrige Natronlauge so schnell hinzu, dass die Temperatur der Reaktionsmischung auf 54° steigt. Es entsteht zunächst eine klare Lösung, aus der alsbald Kristallisation einsetzt. Man lässt unter weiterem Rühren langsam auf Raumtemperatur abkühlen, lässt 8 Stunden bei Raumtemperatur nachrühren, saugt ab, wäscht mit einem Gemisch aus 2 Volumenteilen Methanol und 1 Volumenteil Wasser und trocknet bei 30° unter etwa 20 mbar bis zur Gewichtskonstanz. Man erhält das Dinatrium-3-amino-1-hydroxy-propan-1,1-diphosphonat in der neuen kristallwasserhaltigen Kristallform (Modifikation E), deren IR-Spektrum mit dem des gemäss Beispiel 1 erhaltenen Produktes identisch ist.

Beispiel 5

235 g 3-Amino-1-hydroxy-propan-1,1-diphosphonsäure werden in 2,17 l entmineralisiertem Wasser suspendiert und unter Rühren auf 60°C erwärmt. Dann wird 30%ige wässrige Natronlauge so lang zugegeben, bis der pH-Wert 7,5 beträgt, wofür etwa 200 ml erforderlich sind. Man erwärmt auf 70°, destilliert unter vermindertem Druck etwa 1,2 l Wasser ab, bis Kristallisation einsetzt, und fügt unter Rühren 300 ml Äthanol hinzu. Dann kühlt man unter Rühren auf Raumtemperatur ab, rührt 1 Stunde im Eisbad aus, saugt ab, wäscht mit 400 ml 66%igem Äthanol nach und trocknet unter vermindertem Druck (ca. 20 mbar) bei 120° bis zur Gewichtskonstanz. Man erhält die im Vergleich mit der erfindungsgemässen kristallwasserhaltigen Kristallmodifikation (Modifikation E) wasserärmere, kristalline Modifikation C des Dinatrium-3-amino-1-hydroxy-propan-1,1-

diphosphonates. Diese ist durch folgende Gitterabstände (d-Werte) und relativen Linienintensitäten (Intensitäten) ihres Röntgenpulverdiagramms (Kamera nach Guinier-de-Wolff, Strahlungsquelle: Kupfer-$K_\alpha$) charakterisiert:

| d-Werte (Ångström) | Intensität |
|---|---|
| 10.1 | sehr schwach |
| 9.1 | mittel |
| 8.9 | schwach |
| 6.6 | sehr schwach |
| 5.91 | schwach |
| 5.67 | sehr schwach |
| 5.49 | stark |
| 4.90 | stark |
| 4.73 | mittel |
| 4.54 | mittel |
| 4.19 | schwach |
| 3.61 | sehr schwach |
| 3.13 | schwach |
| 3.11 | schwach |
| 3.06 | schwach |
| 3.03 | sehr schwach |
| 2.98 | schwach |
| 2.91 | schwach |
| 2.86 | mittel |

Beispiel 6

40,0 g Dinatrium-3-amino-1-hydroxy-propan-1,1-diphosphonat in der wasserärmeren Kristallform der Modifikation C werden in 100 ml entmineralisiertem Wasser suspendiert und über Nacht bei Raumtemperatur gerührt. Man lässt noch einige Stunden unter der Mutterlauge im Eisbad stehen, saugt ab, wäscht mit wenig Eiswasser nach und trocknet bei Raumtemperatur unter vermindertem Druck (ca. 20 mbar) bis zur Gewichtskonstanz. Man erhält das Dinatrium-3-amino-1-hydroxy-propan-1,1-diphosphonat in der neuen, kristallwasserhaltigen Kristallform (Modifikation E), deren IR-Spektrum mit dem des gemäss Beispiel 1 erhaltenen Produktes identisch ist.

Beispiel 7

10 kg rohes Dinatrium-3-amino-1-hydroxy-propan-1,1-diphosphonat mit einem Wassergehalt von etwa 7,3 Gew.-% (bestimmt durch Ausheizen auf etwa 180 °C) werden auf Trockenblechen ausgebreitet und in einen Umluftschranktrockner eingebracht, dessen übrige Bleche mit Wasser gefüllt sind. Darauf wird der Trockner in Umluftbetriebsart bei schwächster Gebläseleistungsstufe betrieben, bis das Gewicht der Substanz um etwa 23 bis 26% zugenommen hat. Dann entfernt man die wassergefüllten Bleche und trocknet bei etwa 35° in Mischluftbetriebsart bis zur Gewichtskonstanz. Man erhält das Dinatrium-3-amino-1-hydroxy-propan-1,1-diphosphonat in der neuen, kristallwasserhaltigen Kristallform (Modifikation E), deren IR-Spektrum mit dem des gemäss Beispiel 1 erhaltenen Produktes identisch ist.

Beispiel 8

50,0 g 3-Amino-1-hydroxy-propan-1,1-diphosphonsäure werden in 370 ml entmineralisiertem Wasser suspendiert und unter Rühren auf 50° erwärmt. Dann werden 56,6 g 30%ige wässrige Natronlauge so zugegeben, dass die Temperatur der Reaktionsmischung während der Neutralisation auf 59° steigt. Man erwärmt auf 65°, filtriert heiss über Diatomeenerde, erwärmt auf 75° und destilliert unter Rühren bei vermindertem Druck 300 ml Wasser ab. Man lässt auf etwa 52° abkühlen, impft an und lässt unter Rühren langsam auf Raumtemperatur abkühlen, wobei bei etwa 45° Kristallisation einsetzt. Man lässt über Nacht unter der Mutterlauge stehen, rührt 1 Stunde im Eisbad aus, saugt ab, wäscht mit wenig Eiswasser und trocknet unter vermindertem Druck bei Raumtemperatur bis zur Gewichtskonstanz. Man erhält Dinatrium-3-amino-1-hydroxy-propan-1,1-diphosphonat in Form der im Vergleich mit der erfindungsgemässen kristallwasserhaltigen Kristallmodifikation (Modifikation E) wasserärmere körnig-kristallinen Kristallform der Modifikation B. Diese ist durch folgende Gitterabstände (d-Werte) und relativen Linienintensitäten (Intensitäten) ihres Röntgenpulverdiagramms (Kamera nach Guinier-de-Wolff, Strahlungsquelle: Kupfer-$K_\alpha$) charakterisiert:

| d-Werte (Ångström) | Intensität |
|---|---|
| 11.7 | stark |
| 7.8 | sehr stark |
| 6.8 | sehr stark |
| 5.83 | stark |
| 5.66 | stark |
| 5.38 | sehr schwach |
| 4.76 | mittel |
| 4.66 | mittel |
| 4.29 | sehr schwach |
| 4.20 | sehr schwach |
| 4.13 | schwach |
| 4.06 | mittel |
| 3.99 | mittel |
| 3.89 | mittel |
| 3.82 | stark |
| 3.76 | sehr schwach |
| 3.63 | mittel |
| 3.51 | schwach |
| 3.46 | stark |
| 3.27 | schwach |
| 3.22 | sehr schwach |
| 3.15 | stark |
| 3.08 | stark |
| 3.01 | schwach |
| 2.94 | schwach |
| 2.91 | stark |
| 2.89 | stark |
| 2.86 | schwach |

| d-Werte (Ångström) | Intensität |
|---|---|
| 2.82 | stark |
| 2.78 | mittel |

Das Röntgenpulverdiagramm ist von denjenigen der Modifikation A, C und E verschieden.

Vergleichsbeispiel 1: 2.35 g 3-Amino-1-hydroxy-propan-1,1-diphosphonsäure werden in 100 ml Wasser suspendiert und unter Rühren tropfenweise bis zur vollständigen Neutralisation (pH = 7,4) mit n-Natronlauge (ca. 20,0 ml) versetzt. Man dampft unter vermindertem Druck bei 60–70° zur Trockne ein und trocknet unter vermindertem Druck (ca. 20 mbar) bis zur Gewichtskonstanz. Man erhält Dinatrium-3-amino-1-hydroxy-propan-1,1-diphosphonat in Form eines amorphen, an der Luft zerfliesslichen Produkts, das gemäss Titration nach Karl Fischer etwa 12,9 Gew.-% Wasser aufweist.

Vergleichsbeispiel 2: Man verfährt gemäss Beispiel 8 mit dem Unterschied, dass zur Trocknung des Rohproduktes unter vermindertem Druck bis zur Gewichtskonstanz auf 120° erwärmt wird. Man erhält Dinatrium-3-amino-1-hydroxy-propan-1,1-diphosphonat in weitgehend wasserfreier, kristalliner Form (Modifikation A). Das Produkt ist nach Ausweis des IR-Spektrums identisch mit dem Ausgangsmaterial von Beispiel 1.

Formulierungsbeispiel 1: Gelatinekapseln, enthaltend 200 mg der neuen, kristallwasserhaltigen Kristallmodifikation des Dinatrium-3-amino-1-hydroxy-propan-1,1-diphosphonates (Modifikation E) als Wirkstoff, können z. B. wie folgt hergestellt werden:

| Zusammensetzung (für 1000 Kapseln) | |
|---|---|
| Wirkstoff | 100 g |
| Lactose, gemahlen | 100 g |

Der Wirkstoff und die Lactose (feinst gemahlen) werden gut miteinander vemischt. Das erhaltene Pulver wird gesiebt und in Portionen zu je 0,20 g in Gelatinekapseln abgefüllt.

Formulierungsbeispiel 2: Tabletten, enthaltend 25 mg der neuen, kristallwasserhaltigen Kristallmodifikation des Dinatrium-3-amino-1-hydroxy-propan-1,1-diphosphonates (Modifikation E), können folgendermassen erhalten werden:

| Bestandteile (für 1000 Tabletten) | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Formulierungsbeispiel 3: Tabletten enthaltend 100 mg der neuen, kristallwasserhaltigen Kristallmodifikation des Dinatrium-3-amino-1-hydroxy-propan-1,1-diphosphonates (Modifikation E), können folgendermassen hergestellt werden:

| Bestandteile (für 1000 Tabletten) | |
|---|---|
| Wirkstoff | 100,0 g |
| Lactose | 50,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Formulierungsbeispiel 4: Tabletten enthaltend 75 mg der neuen, kristallwasserhaltigen Kristallmodifikation des Dinatrium-3-amino-1-hydroxy-propan-1,1-diphosphonates (Modifikation E) als Wirkstoff, können folgendermassen hergestellt werden:

| Bestandteile (für 1000 Tabletten) | |
|---|---|
| Wirkstoff | 75,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und

zu beidseitig konkaven Tabletten von etwa 6 mm Durchmesser verpresst.

**Patentansprüche für die Vertragsstaaten: DE, GB, FR, CH, LI, IT, NL, BE, SE, Lu**

1. Kristallines, kristallwasserhaltiges Dinatrium-3-amino-1-hydroxy-propan-1,1-diphosphonat der Formel

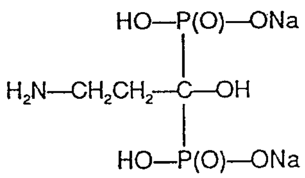

(I)

in nicht-hygroskopischer Form.

2. Dinatrium-3-amino-1-hydroxy-propan-1,1-diphosphonat gemäss Anspruch 1, gekennzeichnet durch einen Wassergehalt von 24,1 bis 24,5 Gew.-%.

3. Dinatrium-3-amino-1-hydroxy-propan-1,1-diphosphonat gemäss Anspruch 1 in Pentahydratform.

4. Dinatrium-3-amino-1-hydroxy-propan-1,1-diphosphonat gemäss Anspruch 1, erhältlich durch Kristallisation aus mindestens gesättigter wässriger Lösung, wobei die Kristallbildung bei 50 bis 80°C und die Trocknung bei 18 bis 25°C oder bei 35 bis 40°C erfolgt, bzw. durch Einwirkung feuchter Luft der relativen Luftfeuchtigkeit von 95 bis 99% bei 20 bis 25°C auf eine feste Erscheinungsform des Dinatrium-3-amino-1-hydroxy-propan-1,1-diphosphonates mit einem Wassergehalt von weniger als 24 Gew.-%.

5. Dinatrium-3-amino-1-hydroxy-propan-1,1-diphosphonat gemäss Anspruch 1, gekennzeichnet durch die nachfolgenden Gitterabstände (d-Werte) und relativen Linienintensitäten (Intensitäten) ihres Röntgenpulverdiagramms (Kamera nach Guinier-de-Wolff, Strahlungsquelle: Kupfer-$K_\alpha$):

| d-Werte (Ångström) | Intensität |
|---|---|
| 10.2 | mittel |
| 9.9 | mittel |
| 9.2 | sehr stark |
| 5.91 | sehr stark |
| 5.57 | stark |
| 5.42 | sehr schwach |
| 5.30 | stark |
| 5.14 | mittel |
| 5.02 | sehr stark |
| 4.97 | sehr stark |
| 4.63 | sehr schwach |
| 4.41 | stark |
| 4.16 | stark |
| 4.07 | schwach |
| 4.04 | mittel |
| 3.95 | sehr schwach |
| 3.75 | stark |
| 3.67 | schwach |
| 3.63 | mittel |
| 3.61 | sehr schwach |
| 5.58 | sehr schwach |
| 3.51 | mittel |
| 3.43 | stark |
| 3.38 | mittel |
| 3.15 | mittel |
| 3.14 | mittel |
| 3.09 | mittel |
| 3.03 | sehr stark |
| 3.01 | sehr stark |
| 2.98 | stark |
| 2.97 | sehr schwach |
| 2.91 | seht stark |
| 2.82 | stark |
| 2.80 | mittel |
| 2.78 | mittel |
| 2.75 | stark |
| 2.73 | schwach |
| 2.71 | schwach |
| 2.69 | schwach |
| 2.67 | mittel |
| 2.66 | mittel |
| 2.63 | stark |
| 2.62 | stark |
| 2.61 | stark |
| 2.58 | sehr stark |
| 2.57 | sehr stark |

6. Für die enterale Verabreichung an Warmblüter bestimmte pharmazeutische Zubereitungen, enthaltend kristallines, kristallwasserhaltiges Dinatrium-3-amino-1-hydroxy-propan-1,1-diphosphonat in nichthygroskopischer Form gemäss einem der Ansprüche 1 bis 5.

7. Verfahren zur Herstellung von kristallinem, kristallwasserhaltigem Dinatrium-3-amino-1-hydroxy-propan-1,1-diphosphonat in nichthygroskopischer Form, dadurch gekennzeichnet, dass man Dinatrium-3-amino-1-hydroxy-propan-1,1-diphosphonat aus wasserhaltiger Lösung zur Kristallisation bringt, wobei man die Kristallbildung bei mindestens 50°C einleitet, bzw. eine feste Erscheinungsform des Dinatrium-3-amino-1-hydroxy-propan-1,1-diphosphonates mit einem Wassergehalt von weniger als 24 Gew.-% mit Wasser behandelt, jeweils das Produkt isoliert und bei 15 bis 60°C trocknet.

8. Verfahren gemäss Anspruch 7, dadurch gekennzeichnet, dass man eine 10%ige bis 55%ige wässrige Lösung von Dinatrium-3-amino-1-hydroxy-propan-1,1-diphosphonat bei 70 bis 80°C bis zur Sättigungskonzentration des Dinatrium-3-amino-1-hydroxy-propan-1,1-diphosphonates einengt und die Kristallisation durch Abkühlen auf 0 bis 5°C bewirkt oder eine 35%- bis 45%ige wässrige Lösung von Dinatrium-3-amino-1-hydroxy-propan-1,1-diphosphonat von anfangs 70 bis 80°C bis zum Einsetzen der Kristallbildung auf nicht weniger als 55°C abkühlt und die Kristallisation durch Abkühlen auf 20 bis 25°C, Hinzufügen von 35 bis 45 Vol-% (bezogen auf die Gesamtwassermenge) Äthanol und Kühlen auf 0 bis 5°C bewirkt.

9. Verfahren zur Herstellung von kristallinem, kristallwasserhaltigem Dinatrium-3-amino-1-hydroxy-propan-1,1-diphosphonat durch Umsetzung von 3-Amino-1-hydroxy-propan-1,1-diphosphonsäure mit einem für deren partielle Neutralisation geeigneten basischen Natriumsalz, dadurch gekennzeichnet, dass man zur Erzielung eines nichthygroskopischen Produktes 3-Amino-1-hydroxy-propan-1,1-diphosphonsäure in wasserhaltiger Suspension und/oder Lösung mit der erforderlichen Menge wässriger Natronlauge umsetzt, das gebildete Dinatrium-3-amino-1-hydroxy-propan-1,1-diphosphonat aus mindestens gesättigter, wasserhaltiger Lösung in Form der gewünschten, kristallwasserhaltigen Form zur Kristallisation bringt, wobei man die Kristallbildung bei mindestens 50°C einleitet, das Produkt isoliert und bei 15 bis 60°C trocknet.

10. Verfahren gemäss Anspruch 9, dadurch gekennzeichnet, dass man die Reaktionsbedingungen so wählt, dass die Neutralisation bei 35 bis 85°C, die Kristallbildung bei 50 bis 70°C und das Kristallwachstum bei 50 bis 70°C erfolgt.

11. Verfahren gemäss Anspruch 7, dadurch gekennzeichnet, dass man eine kristalline Erscheinungsform des Dinatrium-3-amino-1-hydroxy-propan-1,1-diphosphonates mit einem Wassergehalt von weniger als 24 Gew.-% mit mindestens der für die Bildung der neuen, kristallwasserhaltigen Kristallmodifikation erforderlichen und höchstens der dem um 1 verminderten Kehrwert der Sättigungskonzentration des Dinatrium-3-amino-1-hydroxy-propan-1,1-diphosphonates entsprechenden Wassermenge behandelt.

12. Verfahren gemäss Anspruch 11, dadurch gekennzeichnet, dass man mit der 2- bis 3-fachen Gewichtsmenge Wasser verrührt.

13. Verfahren gemäss einem der Ansprüche 7 bis 12, dadurch gekennzeichnet, dass man die Trocknung bei 18 bis 25°C oder bei 35 bis 40°C durchführt.

14. Verfahren nach einem der Ansprüche 9 bis 13, dadurch gekennzeichnet, dass man eine Suspension von 10 Gewichtsteilen 3-Amino-1-hydroxy-propan-1,1-diphosphonsäure in etwa 18 bis 23 Volumenteilen Wasser bei etwa 65 bis etwa 85°C mit der für die Bildung des Dinatrium-3-amino-1-hydroxy-propan-1,1-diphosphonates erforderlichen Menge, 27.5- bis 32,5%iger wässriger Natronlauge neutralisiert, die erhaltene Lösung auf 70 bis 55°C abkühlen lässt, nach Einsetzen oder Auslösen der Kristallbildung auf etwa 20 bis 25°C abkühlt, 7 bis 12 Volumenteile Äthanol hinzugibt, auf 5 bis 0°C weiter abkühlt, bei dieser Temperatur 1 bis 15 Stunden nachrühren lässt, das Produkt durch Abnutschen oder Abzentrifugieren isoliert und in Portionen mit insgesamt 7,5 bis 12,5 Volumenteilen 40- bis 60%igen wässrigen Äthanol nachwäscht oder eine Suspension von 10 Gewichtsteile 3-Amino-1-hydroxy-propan-1,1-diphosphonsäure in 25 bis 37,5 Volumenteilen Wasser bei anfangs 35°C mit der für die Bildung des Dinatrium-3-amino-1-hydroxy-propan-1,1-diphosphonates erforderlichen Menge, 27,5- bis 32,5%iger wässriger Natronlauge neutralisiert, wobei deren Zugabegeschwindigkeit so eingestellt wird, dass die Temperatur auf 50 bis 55°C steigt, nach Einsetzen oder Auslösen der Kristallbildung auf 18°C bis etwa 25°C abkühlen lässt, das Produkt durch Abnutschen isoliert, portionsweise mit insgesamt 15 bis 25 Volumenteilen 50- bis 75%igen wässrigen Äthanols nachwäscht und jeweils bei 35 bis etwa 40°C bis zur Gewichtskonstanz trocknet.

15. Verfahren gemäss einem der Ansprüche 7 bis 14, dadurch gekennzeichnet, dass man unter vermindertem Druck trocknet.

16. Verfahren gemäss einem der Ansprüche 7 bis 15, dadurch gekennzeichnet, dass man bei 5 bis 20 mbar trocknet.

17. Verfahren gemäss Anspruch 7, dadurch gekennzeichnet, dass man auf eine kristalline Erscheinungsform des Dinatrium-3-amino-1-hydroxy-propan-1,1-diphosphonates mit einem Wassergehalt von weniger als 24 Gew.-% bei 20 bis 25°C feuchte Luft der relativen Feuchtigkeit von 90 bis 99% bis zu einer Gewichtszunahme von mindestens etwa 32,2 Gew.-% (unter Berücksichtigung des Ausgangswassergehaltes) einwirken lässt und die Luftfeuchtigkeit dann auf etwa 35 bis etwa 80% senkt.

18. Verfahren gemäss einem der Ansprüche 7, 11, 12 und 17, dadurch gekennzeichnet, dass man als feste Erscheinungsform des Dinatrium-3-amino-1-hydroxy-propan-1,1-diphosphonates mit einem Wassergehalt von weniger als 24 Gew.-% weitgehend wasserfreies Dinatrium-3-amino-1-hydroxy-propan-1,1-diphosphonat oder Dinatrium-3-amino-1-hydroxy-propan-1,1-diphosphonat mit einem Wassergehalt von 5 bis etwa 10 Gew.-%, bestimmt durch Ausheizen auf etwa 180°C, verwendet.

19. Verfahren gemäss einem der Ansprüche 7 bis 18 zur Herstellung eines Dinatrium-3-amino-1-hydroxy-propan-1,1-diphosphonates gemäss einem der Ansprüche 2, 3 und 5.

20. Verwendung der Verbindung gemäss einem der Ansprüche 1 bis 5 bzw. der gemäss einem der Ansprüche 7 bis 19 erhältlichen Verbindung zur Herstellung von für die enterale Verabreichung bestimmten Arzneimittelzubereitungen, bestimmt zur enteralen Behandlung von Störungen des Calcium- und/oder Phosphatstoffwechsels.

21. Dinatrium-3-amino-1-hydroxy-propan-1,1-diphosphonat gemäss Anspruch 1, gekennzeichnet durch die nachfolgenden Gitterabstände (d-Werte) und relativen Linienintensitäten (Intensitäten) ihres Röntgenpulverdiagramms (Kamera nach Guinier-de-Wolff, Strahlungsquelle: Kupfer-$K_{\alpha}$):

| d-Werte (Ångström) | Intensität |
|---|---|
| 10.1 | sehr schwach |

| | |
|---|---|
| 9.1 | mittel |
| 8.9 | schwach |
| 6.6 | sehr schwach |
| 5.91 | schwach |
| 5.67 | sehr schwach |
| 5.49 | stark |
| 4.90 | stark |
| 4.73 | mittel |
| 4.54 | mittel |
| 4.19 | schwach |
| 3.61 | sehr schwach |
| 3.13 | schwach |
| 3.11 | schwach |
| 3.06 | schwach |
| 3.03 | sehr schwach |
| 2.98 | schwach |
| 2.91 | schwach |
| 2.86 | mittel |

22. Verfahren zur Herstellung eines Dinatrium-3-amino-1-hydroxy-propan-1,1-diphosphonates gemäss Anspruch 21, dadurch gekennzeichnet, dass man eine Suspension von 10 Gewichtsteilen 3-Amino-1-hydroxy-propan-1,1-diphosphonsäure in etwa 70 bis 100 Gewichtsteilen Wasser auf 60 °C erwärmt, die für die Bildung des Dinatrium-3-amino-1-hydroxy-propan-1,1-diphosphonates erforderliche Menge 27,5- bis 32,5%iger wässriger Natronlauge hinzugibt, bei 75 °C 50 Gewichtsteile Wasser abdampft, 10 bis 15 Volumenteile Äthanol hinzufügt, auf Raumtemperatur abkühlt, 1 Stunde bei 0 bis 3 °C ausrührt, absaugt mit etwa 60%igem Äthanol wäscht und unter vermindertem Druck bei etwa 120 °C bis zur Gewichtskonstanz trocknet.

23. Dinatrium-3-amino-1-hydroxy-propan-1,1-diphosphonat gemäss Anspruch 1, gekennzeichnet durch die nachfolgenden Gitterabstände (d-Werte) und relativen Linienintensitäten (Intensitäten) ihres Röntgenpulverdiagramms (Kamera nach Guinier-de-Wolff, Strahlungsquelle: Kupfer-$K_\alpha$):

| d-Werte (Ångström) | Intensität |
|---|---|
| 11.7 | stark |
| 7.8 | sehr stark |
| 6.8 | sehr stark |
| 5.83 | stark |
| 5.66 | stark |
| 5.38 | sehr schwach |
| 4.76 | mittel |
| 4.66 | mittel |
| 4.29 | sehr schwach |
| 4.20 | sehr schwach |
| 4.13 | schwach |
| 4.06 | mittel |
| 3.99 | mittel |
| 3.89 | mittel |
| 3.82 | stark |
| 3.76 | sehr schwach |
| 3.63 | mittel |
| 3.51 | schwach |
| 3.46 | stark |
| 3.27 | schwach |
| 3.22 | sehr schwach |
| 3.15 | stark |
| 3.08 | stark |
| 3.01 | schwach |
| 2.94 | schwach |
| 2.91 | stark |
| 2.89 | stark |
| 2.86 | schwach |
| 2.82 | stark |
| 2.78 | mittel |

24. Verfahren zur Herstellung eines Dinatrium-3-amino-1-hydroxy-propan-1,1-diphosphonates gemäss Anspruch 23, dadurch gekennzeichnet, dass man eine wässrige Lösung von Dinatrium-3-amino-1-hydroxy-propan-1,1-diphosphonat bei 75 °C stark einengt, das Produkt durch langsames Abkühlen im Temperaturbereich von 45 bis 0 °C auskristallisieren lässt, abnutscht und bei Raumtemperatur unter vermindertem Druck bis zur Gewichtskonstanz trocknet.

**Patentansprüche für den Vertragsstaat: AT**

1. Verfahren zur Herstellung von kristallinem, kristallwasserhaltigem Dinatrium-3-amino-1-hydroxy-propan-1,1-diphosphonat der Formel

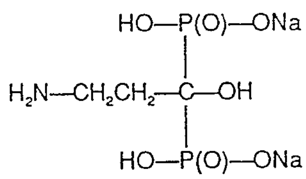

(I)

in nicht-hygroskopischer Form dadurch gekennzeichnet, dass man Dinatrium-3-amino-hydroxy-propan-1,1-diphosphonat aus wasserhaltiger Lösung zur Kristallisation bringt, wobei man die Kristallbildung bei mindestens 50 °C einleitet, bzw. eine feste Erscheinungsform des Dinatrium-3-amino-hydroxy-propan-1,1-diphosphonates mit einem Wassergehalt von weniger als 24 Gew.-% mit Wasser behandelt, jeweils das Produkt isoliert und bei 15 bis 60 °C Temperatur trocknet.

2. Verfahren gemäss Anspruch 1, dadurch gekennzeichnet, dass man eine 10%ige bis 55%ige wässrige Lösung von Dinatrium-3-amino-1-hydroxy-propan-1,1-diphosphonat bei 70 bis 80 °C bis zur Sättigungskonzentration des Dinatrium-3-amino-1-hydroxy-propan-1,1-diphosphonates einengt und die Kristallisation durch Abkühlen auf etwa 0 bis 5 °C bewirkt oder eine 35%- bis 45%ige wässrige Lösung von Dinatrium-3-amino-1-hydroxy-propan-1,1-diphosphonat von anfangs 70 bis 80 °C bis zum Einsetzen der Kristallbildung auf nicht weniger als 55 °C abkühlt und die Kristallisation durch Abkühlen auf 20 bis 25 °C, Hinzufügen von 35 bis 45 Vol-% (bezogen auf die Gesamtwassermenge) Äthanol und Kühlen auf 0 bis 5 °C bewirkt.

3. Verfahren zur Herstellung von kristallinem, kristallwasserhaltigem Dinatrium-3-amino-1-hydroxy-propan-1,1-diphosphonat durch Umsetzung von 3-Amino-1-hydroxy-propan-1,1-diphosphonsäure mit einem für deren partielle Neutralisation geeigneten basischen Natriumsalz, dadurch gekennzeichnet, dass man zur Erzielung eines nichthygroskopischen Produktes 3-Amino-1-hydroxy-propan-1,1-diphosphonsäure in wasserhaltiger Suspension und/oder Lösung mit der erforderlichen Menge wässriger Natronlauge umsetzt, das gebildete Dinatrium-3-amino-1-hydroxy-propan-1,1-diphosphonat aus mindestens gesättigter, wasserhaltiger Lösung in Form der neuen, kristallwasserhaltigen Form zur Kristallisation bringt, das Produkt isoliert und bei 15 bis 60°C trocknet.

4. Verfahren gemäss Anspruch 3, dadurch gekennzeichnet, dass man die Reaktionsbedingungen so wählt, dass die Neutralisation bei 35 bis 85°C die Kristallbildung bei 50 bis 75°C und das Kristallwachstum bei 50 bis 0°C erfolgt.

5. Verfahren gemäss Anspruch 1, dadurch gekennzeichnet, dass man eine feste Erscheinungsform des Dinatrium-3-amino-1-hydroxy-propan-1,1-diphosphonates mit einem Wassergehalt von weniger als 24 Gew.-% mit mindestens der für die Bildung der neuen, kristallwasserhaltigen Kristallmodifikation erforderlichen und höchstens der dem um 1 verminderten Kehrwert der Sättigungskonzentration des Dinatrium-3-amino-1-hydroxy-propan-1,1-diphosphonates entsprechenden Wassermenge behandelt.

6. Verfahren gemäss Anspruch 5, dadurch gekennzeichnet, dass man mit der 2- bis 3-fachen Gewichtsmenge Wasser verrührt.

7. Verfahren gemäss einem der Ansprüche 1 bis 6, dadurch gekennzeichnet, dass man die Trocknung bei 18 bis 25°C oder bei 35 bis 40°C durchführt.

8. Verfahren nach Anspruch 3, 4 oder 7, dadurch gekennzeichnet, dass man eine Suspension von 10 Gewichtsteilen 3-Amino-1-hydroxy-propan-1,1-diphosphonsäure in 18 bis 23 Volumenteilen Wasser bei 65 bis 85°C mit der für die Bildung des Dinatrium-3-amino-1-hydroxy-propan-1,1-diphosphonates erforderlichen Menge 27,5- bis 32,5%iger wässriger Natronlauge neutralisiert, die erhaltene Lösung auf 70 bis 55°C abkühlen lässt, nach Einsetzen oder Auslösen der Kristallbildung auf 20 bis 25°C abkühlt, etwa 7 bis 12 Volumenteile Äthanol hinzugibt, auf 5 bis 0°C weiter abkühlt, bei dieser Temperatur 1 bis 15 Stunden nachrühren lässt, das Produkt durch Abnutschen oder Abzentrifugieren isoliert, in Portionen mit insgesamt 7,5 bis 12,5 Volumenteilen 40- bis 60%igen wässrigen Äthanol nachwäscht oder eine Suspension von 10 Gewichtsteilen 3-Amino-1-hydroxy-propan-1,1-diphosphonsäure in 25 bis 37,5 Volumenteilen Wasser bei anfangs 35°C mit der für die Bildung des Dinatrium-3-amino-1-hydroxy-propan-1,1-diphosphonates erforderlichen Menge 27,5- bis 32,5%iger wässriger Natronlauge neutralisiert, wobei deren Zugabegeschwindigkeit so eingestellt wird, dass die Temperatur auf 50 bis 55°C steigt, nach Einsetzen oder Auslösen der Kristallisation auf 18 bis 25°C abkühlen lässt, das Produkt durch Abnutschen isoliert, portionsweise mit insgesamt 15 bis 25 Volumenteilen 50- bis 75%igen wässrigen Äthanols nachwäscht und jeweils bei 35 bis 40°C bis zur Gewichtskonstanz trocknet.

9. Verfahren gemäss einem der Ansprüche 1 bis 8, dadurch gekennzeichnet, dass man unter vermindertem Druck trocknet.

10. Verfahren gemäss einem der Ansprüche 1 bis 9, dadurch gekennzeichnet, dass man bei 5 bis 20 mbar trocknet.

11. Verfahren gemäss Anspruch 1, dadurch gekennzeichnet, dass man auf eine kristalline Erscheinungsform des Dinatrium-3-amino-1-hydroxy-propan-1,1-diphosphonates mit einem Wassergehalt von weniger als 24 Gew.-% bei 20 bis 25°C feuchte Luft der relativen Feuchtigkeit von 90 bis 99% bis zu einer Gewichtszunahme von mindestens etwa 32,2 Gew.-% (unter Berücksichtigung des Ausgangswassergehaltes) einwirken lässt und die Luftfeuchtigkeit dann auf 35 bis 80% senkt.

12. Verfahren gemäss einem der Ansprüche 1, 5, 6 und 11, dadurch gekennzeichnet, dass man als feste Erscheinungsform des Dinatrium-3-amino-1-hydroxy-propan-1,1-diphosphonates mit einem Wassergehalt von weniger als 24 Gew.-% weitgehend wasserfreies Dinatrium-3-amino-1-hydroxy-propan-1,1-diphosphonat oder Dinatrium-3-amino-1-hydroxy-propan-1,1-diphosphonat mit einem Wassergehalt von 5 bis 10 Gew.-%, bestimmt durch Ausheizen auf etwa 180°C, verwendet.

13. Verfahren gemäss einem der Ansprüche 1 bis 12, dadurch gekennzeichnet, dass man kristallines, kristallwasserhaltiges Dinatrium-3-amino-1-hydroxy-propan-1,1-diphosphonat in nichthygroskopischer Form mit einem Wassergehalt von 24,1 bis 24,5 Gew.-% herstellt.

14. Verfahren gemäss einem der Ansprüche 1 bis 12, dadurch gekennzeichnet, dass man kristallines, kristallwasserhaltiges Dinatrium-3-amino-1-hydroxy-propan-1,1-diphosphonat in nicht-hygroskopischer Pentahydratform herstellt.

15. Verfahren gemäss einem der Ansprüche 1 bis 12, dadurch gekennzeichnet, dass man kristallines, kristallwasserhaltiges Dinatrium-3-amino-1-hydroxy-propan-1,1-diphosphonat in nicht-hygroskopischer Form, gekennzeichnet durch die nachfolgenden Gitterabstände (d-Werte) und relativen Linienintensitäten (Intensitäten) ihres Röntgenpulverdiagramms (Kamera nach Guinier-de-Wolff, Strahlungsquelle: Kupfer-$K_{\alpha}$):

| d-Werte (Ångström) | Intensität |
|---|---|
| 10.2 | mittel |
| 9.9 | mittel |
| 9.2 | sehr stark |
| 5.91 | sehr stark |
| 5.57 | stark |
| 5.42 | sehr schwach |

| | |
|---|---|
| 5.30 | stark |
| 5.14 | mittel |
| 5.02 | sehr stark |
| 4.97 | sehr stark |
| 4.63 | sehr schwach |
| 4.41 | stark |
| 4.16 | stark |
| 4.07 | schwach |
| 4.04 | mittel |
| 3.95 | sehr schwach |
| 3.75 | stark |
| 3.67 | schwach |
| 3.63 | mittel |
| 3.61 | sehr schwach |
| 5.58 | sehr schwach |
| 3.51 | mittel |
| 3.43 | stark |
| 3.38 | mittel |
| 3.15 | mittel |
| 3.14 | mittel |
| 3.09 | mittel |
| 3.03 | sehr stark |
| 3.01 | sehr stark |
| 2.98 | stark |
| 2.97 | sehr schwach |
| 2.91 | sehr stark |
| 2.82 | stark |
| 2.80 | mittel |
| 2.78 | mittel |
| 2.75 | stark |
| 2.73 | schwach |
| 2.71 | schwach |
| 2.69 | schwach |
| 2.67 | mittel |
| 2.66 | mittel |
| 2.63 | stark |
| 2.62 | stark |
| 2.61 | stark |
| 2.58 | sehr stark |
| 2.57 | sehr stark |

16. Verfahren zur Herstellung für die enterale Verabreichung an Warmblüter bestimmter pharmazeutischer Zubereitungen, enthaltend Dinatrium-3-amino-1-hydroxy-propan-1,1-diphosphonat, dadurch gekennzeichnet, dass man Dinatrium-3-amino-1-hydroxy-propan-1,1-diphosphonat in gemäss einem der Ansprüche 1 bis 16 erhältlicher nichthygroskopischer, kristalliner, kristallwasserhaltiger Form mit üblichen pharmazeutischen Hilfsstoffen vermischt.

17. Verfahren zur Herstellung von kristallinem, kristallwasserhaltigem Dinatrium-3-amino-1-hydroxy-propan-1,1-diphosphonat, gekennzeichnet durch die nachfolgenden Gitterabstände (d-Werte) und relativen Linienintensitäten (Intensitäten) ihres Röntgenpulverdiagramms (Kamera nach Guinier-de-Wolff, Strahlungsquelle: Kupfer-$K_\alpha$):

| d-Werte (Ångström) | Intensität |
|---|---|
| 10.1 | sehr schwach |
| 9.1 | mittel |
| 8.9 | schwach |
| 6.6 | sehr schwach |
| 5.91 | schwach |
| 5.67 | sehr schwach |
| 5.49 | stark |
| 4.90 | stark |
| 4.73 | mittel |
| 4.54 | mittel |
| 4.19 | schwach |
| 3.61 | sehr schwach |
| 3.13 | schwach |
| 3.11 | schwach |
| 3.06 | schwach |
| 3.03 | sehr schwach |
| 2.98 | schwach |
| 2.91 | schwach |
| 2.86 | mittel |

dadurch gekennzeichnet, dass man eine Suspension von 10 Gewichtsteilen 3-Amino-1-hydroxy-propan-1,1-diphosphonsäure in etwa 70 bis 100 Gewichtsteilen Wasser auf 60 °C erwärmt, die für die Bildung des Dinatrium-3-amino-1-hydroxy-propan-1,1-diphosphonates erforderliche Menge 27,5- bis 32,5%iger wässriger Natronlauge hinzugibt, bei 75 °C 50 Gewichtsteile Wasser abdampft, 10 bis 15 Volumenteile Äthanol hinzufügt, auf Raumtemperatur abkühlt, 1 Stunde bei 0 bis 3°C ausrührt, absaugt mit etwa 60%igem Äthanol wäscht und unter vermindertem Druck bei etwa 120 °C bis zur Gewichskonstanz trocknet.

18. Verfahren zur Herstellung von kristallinem, kristallwasserhaltigem Dinatrium-3-amino-1-hydroxy-propan-1,1-diphosphonat, gekennzeichnet durch die nachfolgenden Gitterabstände (d-Werte) und relativen Linienintensitäten (Intensitäten) ihres Röntgenpulverdiagramms (Kamera nach Guinier-de-Wolff, Strahlungsquelle: Kupfer-$K_\alpha$):

| d-Werte (Ångström) | Intensität |
|---|---|
| 11.7 | stark |
| 7.8 | sehr stark |
| 6.8 | sehr stark |
| 5.83 | stark |
| 5.66 | stark |
| 5.38 | sehr schwach |
| 4.76 | mittel |
| 4.66 | mittel |
| 4.29 | sehr schwach |
| 4.20 | sehr schwach |
| 4.13 | schwach |
| 4.06 | mittel |
| 3.99 | mittel |
| 3.89 | mittel |
| 3.82 | stark |
| 3.76 | sehr schwach |
| 3.63 | mittel |
| 3.51 | schwach |
| 3.46 | stark |
| 3.27 | schwach |

| | |
|---|---|
| 3.22 | sehr schwach |
| 3.15 | stark |
| 3.08 | stark |
| 3.01 | schwach |
| 2.94 | schwach |
| 2.91 | stark |
| 2.89 | stark |
| 2.86 | schwach |
| 2.82 | stark |
| 2.78 | mittel |

dadurch gekennzeichnet, dass man eine wässrige Lösung von Dinatrium-3-amino-1-hydroxypropan-1,1-diphosphonat bei 75°C stark einengt, das Produkt durch langsames Abkühlen im Temperaturbereich von 45 bis 0°C auskristallisieren lässt, abnutscht und bei Raumtemperatur unter vermindertem Druck bis zur Gewichtskonstanz trocknet.

**Claims for the Contracting States: DE, GB, FR, CH, LI, IT, NL, BE, SE, LU**

1. Crystalline disodium 3-amino-1-hydroxypropane-1,1-diphosphonate of the formula

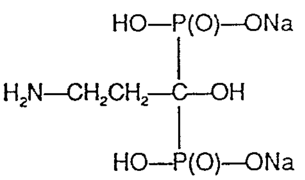

(I)

that contains water of crystallisation and is in nonhygroscopic form.

2. Disodium 3-amino-1-hydroxypropane-1,1-diphosphonate according to claim 1, characterised by a water content of from 24.1 to 24.5% by weight.

3. Disodium 3-amino-1-hydroxypropane-1,1-diphosphonate according to claim 1 in pentahydrate form.

4. Disodium 3-amino-1-hydroxypropane-1,1-diphosphonate according to claim 1, obtainable by crystallisation from an at least saturated aqueous solution, crystal formation taking place at from 50 to 80°C, and drying at from 18 to 25°C or at from 35 to 40°C, or by the action of moist air having a relative atmospheric humidity of from 95 to 99°C at from 20 to 25°C on a solid form of disodium 3-amino-1-hydroxypropane-1,1-diphosphonate having a water content of less than 24% by weight.

5. Disodium 3-amino-1-hydroxypropane-1,1-diphosphonate according to claim 1, characterised by the following lattice spacings (d-values) and relative line intensities (intensities) of its X-ray powder pattern (camera according to Guinier-de-Wolff, radiation source: copper-$K_\alpha$):

| d-values (Ångström) | Intensity |
|---|---|
| 10.2 | medium |
| 9.9 | medium |
| 9.2 | very strong |
| 5.91 | very strong |
| 5.57 | strong |
| 5.42 | very weak |
| 5.30 | strong |
| 5.14 | medium |
| 5.02 | very strong |
| 4.97 | very strong |
| 4.63 | very weak |
| 4.41 | strong |
| 4.16 | strong |
| 4.07 | weak |
| 4.04 | medium |
| 3.95 | very weak |
| 3.75 | strong |
| 3.67 | weak |
| 3.63 | medium |
| 3.61 | very weak |
| 3.58 | very weak |
| 3.51 | medium |
| 3.43 | strong |
| 3.38 | medium |
| 3.15 | medium |
| 3.14 | medium |
| 3.09 | medium |
| 3.03 | very strong |
| 3.01 | very strong |
| 2.98 | strong |
| 2.97 | very weak |
| 2.91 | very strong |
| 2.82 | strong |
| 2.80 | medium |
| 2.78 | medium |
| 2.75 | strong |
| 2.73 | weak |
| 2.71 | weak |
| 2.69 | weak |
| 2.67 | medium |
| 2.66 | medium |
| 2.63 | strong |
| 2.62 | strong |
| 2.61 | strong |
| 2.58 | very strong |
| 2.57 | very strong |

6. Pharmaceutical preparations for enteral administration to warm-blooded animals, containing crystalline disodium 3-amino-1-hydroxypropane-1,1-diphosphonate that contains water of crystallisation and is in non-hygroscopic form according to any one of claims 1 to 5.

7. Process for the manufacture of crystalline disodium 3-amino-1-hydroxypropane-1,1-diphosphonate that contains water of crystallisation and is in nonhygroscopic form, characterised in that disodium 3-amino-1-hydroxypropane-1,1-diphosphonate is caused to crystallise from a water-containing solution, crystal formation being initiated at at least 50°C, or that a solid form of diphosphonate having a water content of less than 24% by weight is treated with water, and in each case the product is isolated and dried at from 15 to 60°C.

8. Process according to claim 7, characterised

in that a 10 to 55% aqueous solution of disodium 3-amino-1-hydroxypropane-1,1-diphosphonate is concentrated at from 70 to 80°C to the saturation concentration of disodium 3-amino-1-hydroxypropane-1,1-diphosphonate and crystallisation is effected by cooling to from 0 to 5°C or that a 35 to 45% aqueous solution of disodium 3-amino-1-hydroxypropane-1,1-diphosphonate is cooled from an initial temperature of from 70 to 80°C to not less than 55°C until the start of crystal formation, and crystallisation is effected by cooling to from 20 to 25°C, adding from 35 to 45% by volume (based on the total amount of water) of ethanol and cooling to from 0 to 5°C.

9. Process for the manufacture of crystalline disodium 3-amino-1-hydroxypropane-1,1-diphosphonate that contains water of crystallisation by reacting 3-amino-1-hydroxypropane-1,1-diphosphonic acid with a basic sodium salt suitable for the partial neutralisation thereof, characterised in that in order to obtain a non-hygroscopic product 3-amino-1-hydroxypropane-1,1-diphosphonic acid in a water-containing suspension and/or solution is reacted with the requisite amount of aqueous sodium hydroxide solution, the resulting disodium 3-amino-1-hydroxypropane-1,1-diphosphonate is caused to crystallise from an at least saturated water-containing solution in the form of the desired form containing water of crystallisation, crystal formation being initiated at at least 50°C, and the product is isolated and dried at from 15 to 60°C.

10. Process according to claim 9, characterised in that the reaction conditions are so selected that neutralisation takes place at from 35 to 85°C, crystal formation takes place at from 50 to 70°C, and crystal growth takes place at from 50 to 0°C.

11. Process according to claim 7, characterised in that a crystalline form of disodium 3-amino-1-hydroxypropane-1,1-diphosphonate having a water content of less than 24% by weight is treated with at least that amount of water that is required for the formation of the novel crystal modification containing water of crystallisation and at most that amount of water that corresponds to the reciprocal value, reduced by 1, of the saturation concentration of disodium 3-amino-1-hydroxypropane-1,1-diphosphonate.

12. Process according to claim 11, characterised in that stirring is carried out with from 2 to 3 times the amount by weight of water.

13. Process according to any one of claims 7 to 12, characterised in that drying is carried out at from 18 to 25°C or at from 35 to 40°C.

14. Process according to any one of claims 9 to 13, characterised in that a suspension of 10 parts by weight of 3-amino-1-hydroxypropane-1,1-diphosphonic acid in approximately from 18 to 23 parts by volume of water is neutralised at from approximately 65°C to approximately 85°C with that amount of 27.5 to 32.5% aqueous sodium hydroxide solution that is required for the formation of disodium 3-amino-1-hydroxypropane-1,1-diphosphonate, the resulting solution is allowed to cool to from 70 to 55°C and, when crystal formation has begun or been initiated, cooled to approximately from 20 to 25°C, from 7 to 12 parts by volume of ethanol are added, the whole is cooled further to from 5 to 0°C and then stirred at this temperature for approximately from 1 to 15 hours, and the product is isolated by filtering off with suction or centrifuging and subsequently washed in portions with a total of from 7.5 to 12.5 parts by volume of from 40 to 60% aqueous ehtanol, or a suspension of 10 parts by weight of 3-amino-1-hydroxypropane-1,1-diphosphonic acid in from 25 to 37.5 parts by volume of water is neutralised at an initial temperature of 35°C with that amount of from 27.5 to 32.5% aqueous sodium hydroxide solution that is required for the formation of disodium 3-amino-1-hydroxypropane-1,1-diphosphonate, the rate of addition thereof being so adjusted that the temperature rises to from 50 to 55°C and, when crystal formation has begun or been initiated, the whole is allowed to cool to from 18 to approximately 25°C, and the product is isolated by filtering off with suction, subsequently washed in portions with a total of from 15 to 25 parts by volume of from 50 to 75% aqueous ethanol and dried in each case at from 35°C to approximately 40°C until the weight is constant.

15. Process according to any one of claims 7 to 14, characterised in that drying is effected under reduced pressure.

16. Process according to any one of claims 7 to 15, characterised in that drying is effected at from 5 to 20 mbar.

17. Process according to claim 7, characterised in that moist air having a relative humidity of from 90 to 99% is allowed to act on a crystalline form of disodium 3-amino-1-hydroxypropane--1,1-diphosphonate having a water content of less than 24% by weight, at from 20 to 25°C, until there is a weight increase of at least approximately 32.2% by weight (taking into consideration the initial water content), and the atmospheric humidity is then reduced to from approximately 35% to approximately 80%.

18. Process according to any one of claims 7, 11, 12, and 17, characterised in that as the solid form of disodium 3-amino-1-hydroxypropane-1,1-diphosphonate having a water content of less than 24% by weight there is used substantially anhydrous disodium 3-amino-1-hydroxypropane-1,1-diphosphonate, or disodium 3-amino-1-hydroxypropane-1,1-diphosphonate having a water content of from 5% to approximately 10% by weight, determined by removing by heating at approximately 180°C.

19. Process according to any one of claims 7 to 18 for the manufacture of a disodium 3-amino-1-hydroxypropane-1,1-diphosphonate according to any one of claims 2, 3 and 5.

20. Use of compound according to any one of claims 1 to 5 or a compound obtainable according to any one of claims 7 to 19 for the manufacture of medicinal preparations intended for enteral administration, which are intended for the enteral

treatment of disorders of the calcium and/or phosphate metabolism.

21. Disodium 3-amino-1-hydroxypropane-1,1-diphosphonate according to claim 1, characterised by the following lattice spacings (d-values) and relative line intensities (intensities) of its X-ray powder pattern (camera according to Guinier-de-Wolff, radiation source: copper-$K_\alpha$):

| d-values (Ångström) | Intensity |
|---|---|
| 10.1 | very weak |
| 9.1 | medium |
| 8.9 | weak |
| 6.6 | very weak |
| 5.91 | weak |
| 5.67 | very weak |
| 5.49 | strong |
| 4.90 | strong |
| 4.73 | medium |
| 4.54 | medium |
| 4.19 | weak |
| 3.61 | very weak |
| 3.13 | weak |
| 3.11 | weak |
| 3.06 | weak |
| 3.03 | very weak |
| 2.98 | weak |
| 2.91 | weak |
| 2.86 | medium |

22. Process for the manufacture of a disodium 3-amino-1-hydroxypropane-1,1-diphosphonate according to claim 21, characterised in that a suspension of 10 parts by weight of 3-amino-1-hydroxy-propane-1,1-diphosphonic acid in approximately from 70 to 100 parts by weight of water is heated to 60 °C, that amount of from 27.5 to 32.5% aqueous sodium hydroxide solution that is required for the formation of disodium 3-amino-1-hydroxypropane-1,1-diphosphonate is added, 50 parts by weight of water are evaporated off at 75 °C, from 10 to 15 parts by volume of ethanol are added, and the whole is cooled to room temperature, stirred for 1 hour at from 0 to 3 °C to complete the reaction, filtered with suction, washed with approximately 60% ethanol and dried under reduced pressure at approximately 120 °C until the weight is constant.

23. Disodium 3-amino-1-hydroxypropane-1,1-diphosphonate according to claim 1, characterised by the following lattice spacings (d-values) and relative line intensities (intensities) of its X-ray powder pattern (camera according to Guinier-de-Wolff, radiation source: copper-$K_\alpha$):

| d-values (Ångström) | Intensity |
|---|---|
| 11.7 | strong |
| 7.8 | very strong |
| 6.8 | very strong |
| 5.83 | strong |
| 5.66 | strong |
| 5.38 | very weak |
| 4.76 | medium |
| 4.66 | medium |
| 4.29 | very weak |
| 4.20 | very weak |
| 4.13 | weak |
| 4.06 | medium |
| 3.99 | medium |
| 3.89 | medium |
| 3.82 | strong |
| 3.76 | very weak |
| 3.63 | medium |
| 3.51 | weak |
| 3.46 | strong |
| 3.27 | weak |
| 3.22 | very weak |
| 3.15 | strong |
| 3.08 | strong |
| 3.01 | weak |
| 2.94 | weak |
| 2.91 | strong |
| 2.89 | strong |
| 2.86 | weak |
| 2.82 | strong |
| 2.78 | medium |

24. Prosess for the manufacture of a disodium 3-amino-1-hydroxypropane-1,1-diphosphonate according to claim 23, characterised in that an aqueous solution of disodium 3-amino-1-hydroxypropane-1,1-diphosphonate is extensively concentrated at 75 °C, the product is allowed to crystallise out by slow cooling in the temperature range of from 45 to 0 °C, filtered off with suction and dried at room temperature under reduced pressure until the weight is constant.

**Claims for the Contracting State: AT**

1. Process for the manufacture of crystalline disodium 3-amino-1-hydroxypropane-1,1-diphosphonate of the formula

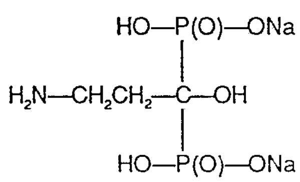

(I)

that contains water of crystallisation and is in non-hygroscopic form, characterised in that disodium 3-amino-1-hydroxypropane-1,1-diphosphonate is caused to crystallise from a water-containing solution, crystal formation being initiated at at least 50 °C, or that a solid form of disodium 3-amino-1-hydroxypropane-1,1-diphosphonate have a water content of less than 24% by weight is treated with water, and in each case the product is isolated and dried at a temperature of from 15 to 60 °C.

2. Process according to claim 1, characterised

in that a 10 to 55% aqueous solution of disodium 3-amino-1-hydroxypropane-1,1-diphosphonate is concentrated at from 70 to 80°C to the saturation concentration of disodium 3-amino-1-hydroxypropane-1,1-diphosphonate and crystallisation is effected by cooling to approximately from 0 to 5°C, or a 35 to 45% aqueous solution of disodium 3-amino-1-hydroxypropane-1,1-diphosphonate is cooled from an initial temperature of from 70 to 80°C to not less than 55°C until the start of crystal formation, and crystallisation is effected by cooling to from 20 to 25°C, adding from 35 to 45% by volume (based on the total amount of water) of ethanol and cooling to from 0 to 5°C.

3. Process for the manufacture of crystalline disodium 3-amino-1-hydroxypropane-1,1-diphosphonate that contains water of crystallisation by reacting 3-amino-1-hydroxypropane-1,1-diphosphonic acid with a basic sodium salt suitable for the partial neutralisation thereof, characterised in that in order to obtain a non-hygroscopic product 3-amino-1-hydroxypropane-1,1-disphosphonic acid in a water-containing suspension and/or solution is reacted with the requisite amount of aqueous sodium hydroxide solution, the resulting disodium 3-amino-1-hydroxypropane-1,1-diphosphonate is caused to crystallise from an at least saturated water-containing solution in the form of the novel form containing water of crystallisation, and the product is isolated and dried at from 15 to 60°C.

4. Process according to claim 3, characterised in that the reaction conditions are so selected that neutralisation takes place at from 35 to 85°C crystal formation takes place at from 50 to 75°C, and crystal growth takes place at from 50 to 0°C.

5. Process according to claim 1, characterised in that a solid form of disodium 3-amino-1-hydroxypropane-1,1-diphosphonate having a water content of less than 24% by weight is treated with at least that amount of water that is required for the formation of the novel crystal modification containing water of crystallisation and at most that amount of water that corresponds to the reciprocal value, reduced by 1, of the saturation concentration of disodium 3-amino-1-hydroxypropane-1,1-diphosphonate.

6. Process according to claim 5, characterised in that stirring is carried out with from 2 to 3 times the amount by weight of water.

7. Process according to any one of claims 1 to 6, characterised in that drying is carried out at from 18 to 25°C or at from 35 to 40°C.

8. Process according to claim 3, 4, or 7, characterised in that a suspension of 10 parts by weight of 3-amino-1-hydroxypropane-1,1-diphosphonic acid in from 18 to 23 parts by volume of water is neutralised at from 65 to 85°C with that amount of 27.5 to 32,5% aqueous sodium hydroxide solution that is required for formation of disodium 3-amino-1-hydroxypropane-1,1-diphosphonate, the resulting solution is allowed to cool to form 70 to 55°C and, when crystal formation has begun or been initiated, cooled to from 20 to 25°C, approximately from 7 to 12 parts by volume of ethanol are added, the whole is cooled further to from 5 to 0°C and then stirred at this temperature for approximately from 1 to 15 hours, and the product is isolated by filtering off with suction or centrifuging and subsequently washed in portions with a total of from 7.5 to 12.5 parts by volume of from 40 to 60% aqueous ethanol, or a suspension of 10 parts by weight of 3-amino-1-hydroxypropane-1,1-diphosphonic acid in from 25 to 37.5 parts by volume of water is neutralised at an initial temperature of 35°C with that amount of from 27.5 to 32.5% aqueous sodium hydroxide solution that is required for the formation of disodium 3-amino-1-hydroxypropane-1,1-diphosphonate, the rate of addition thereof being so adjusted that the temperature rises to from 50 to 55°C and, when crystallisation has begun or been initiated, the whole is allowed to cool to from 18 to 25°C, and the product is isolated by filtering off with suction, subsequently washed in portions with a total of from 15 to 25 parts by volume of from 50 to 75% aqueous ethanol and dried in each case at from 35 to 40°C until the weight is constant.

9. Process according to any one of claims 1 to 8, characterised in that drying is effected under reduced pressure.

10. Process according to any one of claims 1 to 9, characterised in that drying is effected at from 5 to 20 mbar.

11. Process according to claim 1, characterised in that moist air having a relative humidity of from 90 to 99% is allowed to act on a crystalline form of disodium 3-amino-1-hydroxypropane-1,1-diphosphonate having a water content of less than 24% by weight, at from 20 to 25°C, until there is a weight increase of at least approximately 32.2% by weight (taking into consideration the initial water content), and the atmospheric humidity is then reduced to from 35 to 80%.

12. Process according to any one of claims 1, 5, 6, and 11, characterised in that as the solid form of disodium 3-amino-1-hydroxypropane-1,1-diphosphonate having a water content of less than 24% by weight there is used substantially anhydrous disodium 3-amino-1-hydroxypropane-1,1-diphosphonate or disodium 3-amino-1-hydroxypropane-1,1-diphosphonate having a water content of from 5 to 10% by weight, determined by removing by heating at approximately 180°C.

13. Process according to any one of claims 1 to 12, characterised in that there is manufactured crystalline disodium 3-amino-1-hydroxypropane-1,1-diphosphonate that contains water of crystallisation and is in non-hygroscopic form having a water content of from 24.1 to 24.5% by weight.

14. Process according to any one of claims 1 to 12, characterised in that there is manufactured crystalline disodium 3-amino-1-hydroxypropane-1,1-diphosphonate that contains water of crystallisation and is in non-hygroscopic pentahydrate form.

15. Process according to any one of claims 1 to 12, characterised in that there is manufactured crystalline disodium 3-amino-1-hydroxypropane-1,1-diphosphonate that contains water of crystallisation and is in non-hygroscopic form and that is characterised by the following lattice spacings (d-values) and relative line intensities (intensities) of its X-ray powder pattern (camera according to Guinier-de-Wolff, radiation source: copper-$K_\alpha$):

| d-values (Ångström) | Intensity |
|---|---|
| 10.2 | medium |
| 9.9 | medium |
| 9.2 | very strong |
| 5.91 | very strong |
| 5.57 | strong |
| 5.42 | very weak |
| 5.30 | strong |
| 5.14 | medium |
| 5.02 | very strong |
| 4.97 | very strong |
| 4.63 | very weak |
| 4.41 | strong |
| 4.16 | strong |
| 4.07 | weak |
| 4.04 | medium |
| 3.95 | very weak |
| 3.75 | strong |
| 3.67 | weak |
| 3.63 | medium |
| 3.61 | very weak |
| 5.58 | very weak |
| 3.51 | medium |
| 3.43 | strong |
| 3.38 | medium |
| 3.15 | medium |
| 3.14 | medium |
| 3.09 | medium |
| 3.03 | very strong |
| 3.01 | very strong |
| 2.98 | strong |
| 2.97 | very weak |
| 2.91 | very strong |
| 2.82 | strong |
| 2.80 | medium |
| 2.78 | medium |
| 2.75 | strong |
| 2.73 | weak |
| 2.71 | weak |
| 2.69 | weak |
| 2.67 | medium |
| 2.66 | medium |
| 2.63 | strong |
| 2.62 | strong |
| 2.58 | very strong |
| 2.57 | very strong |

16. Prozess for the manufacture of pharmaceutical preparations intended for enteral administration to warm-blooded animals, containing disodium 3-amino-1-hydroxypropane-1,1-diphosphonate, characterised in that disodium 3-amino-1-hydroxypropane-1,1-diphosphonate in non-hygroscopic crystalline form that contains water of crystallisation and is obtainable in accordance with any one of claims 1 to 16 is mixed with customary pharmaceutical auxiliaries.

17. Process for the manufacture of crystalline disodium 3-amino-1-hydroxypropane-1,1-diphosphonate that contains water of crystallisation, characterised by the following lattice spacings (d-values) and relative line intensities (intensities) of its X-ray powder pattern (camera according to Guinier-de-Wolff, radiation source: copper-$K_\alpha$):

| d-values (Ångström) | Intensity |
|---|---|
| 10.1 | very weak |
| 9.1 | medium |
| 8.9 | weak |
| 6.6 | the very weak |
| 5.91 | weak |
| 5.67 | very weak |
| 5.49 | strong |
| 4.90 | strong |
| 4.73 | medium |
| 4.54 | medium |
| 4.19 | weak |
| 3.61 | very weak |
| 3.13 | weak |
| 3.11 | weak |
| 3.06 | weak |
| 3.03 | very weak |
| 2.98 | weak |
| 2.91 | weak |
| 2.86 | medium |

characterised in that a suspension of 10 parts by weight of 3-amino-1-hydroxypropane-1,1-diphosphonic acid in approximately from 70 to 100 parts by weight of water is heated to 60 °C, that amount of 27.5 to 32.5% aqueous sodium hydroxide solution that is required for the formation of disodium 3-amino-1-hydroxypropane-1,1-diphosphonate is added, 50 parts by weight of water are evaporated off at 75 °C, from 10 to 15 parts by volume of ethanol are added, and the whole is cooled to room temperature, stirred for 1 hour at from 0 to 3 °C to complete the reaction, filtered with suction, washed with approximately 60% ethanol, and dried under reduced pressure at approximately 120 °C until the weight is constant.

18. Process for the manufacture of crystalline disodium 3-amino-1-hydroxypropane-1,1-diphosphonate that contains water of crystallisation, characterised by the following lattice spacings (d-values) and relative line intensities (intensities) of its X-ray powder pattern (camera according to Guinier-de-Wolff, radiation source: copper-$K_\alpha$):

| | |
|---|---|
| 11.7 | strong |
| 7.8 | very strong |
| 6.8 | very strong |
| 5.83 | strong |
| 5.66 | strong |
| 5.38 | very weak |
| 4.76 | medium |
| 4.66 | medium |
| 4.29 | very weak |
| 4.20 | very weak |
| 4.13 | weak |
| 4.06 | medium |
| 3.99 | medium |
| 3.89 | medium |
| 3.82 | strong |
| 3.76 | very weak |
| 3.63 | medium |
| 3.51 | weak |
| 3.46 | strong |
| 3.27 | weak |
| 3.22 | very weak |
| 3.15 | strong |
| 3.08 | strong |
| 3.01 | weak |
| 2.94 | weak |
| 2.91 | strong |
| 2.89 | strong |
| 2.86 | weak |
| 2.82 | strong |
| 2.78 | medium |

characterised in that an aqueous solution of disodium 3-amino-1-hydroxypropane-1,1-diphosphonate is extensively concentrated at 75°C, the product is allowed to crystallise out by slow cooling in the temperature range of from 45 to 0°C, filtered off with suction and dried at room temperature under reduced pressure until the weight is constant.

**Revendications pour les Etats contractants: DE, GB, FR, CH, LI, IT, NL, BE, SE, LU**

1. 3-amino-1-hydroxypropane-1,1-diphosphonate disodique de formule

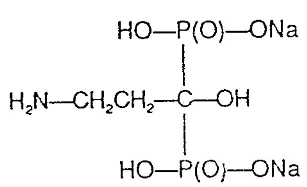

(I)

sous forme non hygroscopique.

2. 3-amino-1-hydroxypropane-1,1-diphosphonate disodique selon la revendication 1, caractérisé par une teneur en eau de 24,1 à 24,5% en poids.

3. 3-amino-1-hydroxypropane-1,1-diphosphonate disodique selon la revendication 1 sous forme de pentahydrate.

4. 3-amino-1-hydroxypropane-1,1-diphosphonate disodique selon la revendication 1, obtenu par cristallisation à partir d'une solution aqueuse saturée, la cristallisation ayant lieu entre 50 et 80°C et le séchage entre 18 et 25°C, ou entre 35 et 40°C, ou bien par action d'air humide, d'humidité relative de 95 à 99% entre 20 et 25°C sur une forme solide de 3-amino-1-hydroxypropane-1,1-diphosphonate disodique avec une teneur en eau inférieure à 24% en poids.

5. 3-amino-1-hydroxypropane-1,1-diphosphonate disodique selon la revendication 1, caractérisé par les paramètres de réseau suivant (valeurs d) et par les intensités relatives de raies (intensités) de son diagramme de poudre de rayons X (chambre Guinier-de-Wolff, source de rayonnement raie du cuivre $K_{\alpha}$):

| Valeurs d (Ångström) | Intensité |
|---|---|
| 10,2 | moyenne |
| 9,9 | moyenne |
| 9,2 | très forte |
| 5,91 | très forte |
| 5,57 | forte |
| 5,42 | très faible |
| 5,30 | forte |
| 5,14 | moyenne |
| 5,02 | très forte |
| 4,97 | très forte |
| 4,63 | très faible |
| 4,41 | forte |
| 4,16 | forte |
| 4,07 | faible |
| 4,04 | moyenne |
| 3,95 | très faible |
| 3,75 | forte |
| 3,67 | faible |
| 3,63 | moyenne |
| 3,61 | très faible |
| 3,58 | très faible |
| 3,51 | moyenne |
| 3,43 | forte |
| 3,38 | moyenne |
| 3,15 | moyenne |
| 3,14 | moyenne |
| 3,09 | moyenne |
| 3,03 | très forte |
| 3,01 | très forte |
| 2,98 | forte |
| 2,97 | très faible |
| 2,91 | très forte |
| 2,82 | forte |
| 2,80 | moyenne |
| 2,78 | moyenne |
| 2,75 | forte |
| 2,73 | faible |
| 2,71 | faible |
| 2,69 | faible |
| 2,67 | moyenne |
| 2,66 | moyenne |
| 2,63 | forte |
| 2,62 | forte |
| 2,61 | forte |
| 2,58 | très forte |
| 2,57 | très forte |

6. Préparations pharmaceutiques destinées à l'administration entérale aux êtres à sang chaud comprenant du 3-Amino-1-hydroxypropane-1,1-diphosphonate disodique cristallin, avec eau de cristallisation sous forme non hygroscopique selon l'une des revendications 1 à 5.

7. Procédé de préparation de 3-amino-1-hydroxypropane-1,1-diphosphonate disodique cristallin, avec eau de cristallisation, sous forme non hygroscopique, caractérisé en ce qu'on fait cristalliser du 3-amino-1-hydroxypropane-1,1-diphosphonate disodique à partir de solution aqueuse, en amorçant la cristallisation à au moins 50°C, ou bien on traite avec de l'eau une forme solide de 3-amino-1-hydroxypropane-1,1-diphosphonate disodique avec une teneur en eau inférieure à 24%, on isole chaque fois le produit et on sèche entre 15 et 60°C.

8. Procédé selon la revendication 7, caractérisé en ce qu'on concentre une solution aqueuse de 10 à 55% de 3-amino-1-hydroxypropane-1,1-diphosphonate disodique entre 70 et 80°C jusqu'à concentration de saturation du 3-amino-1-hydroxypropane-1,1-diphosphonate disodique et on provoque la cristallisation par refroidissement entre 0 et 5°C, ou on refroidit une solution aqueuse de 35 à 45% de 3-amino-1-hydroxypropane-1,1-diphosphonate disodique, entre 70 et 80°C au départ, jusqu'à une température qui n'est pas inférieure à 55°C pour commencer la cristallisation, et on provoque la cristallisation par refroidissement entre 20 et 25°C, et addition de 35 à 45% en volume d'éthanol (par rapport à la quantité totale d'eau) et on refroidit entre 0 et 5°C.

9. Procédé de préparation de 3-amino-1-hydroxy-propane-1,1-diphosphonate disodique cristallin comprenant de l'eau de cristallisation par réaction d'acid 3-amino-1-hydroxypropane-1,1-diphosphonique avec un sel basique de sodium approprié pour sa neutralisation partielle, caractérisé en ce que pour obtenir un produit non hygroscopique d'acide 3-amino-1-hydroxypropane-1,1-diphosphonique en suspension aqueuse et/ou solution on fait réagir avec la quantité nécessaire de solution aqueuse de soude, on fait cristalliser le 3-amino-1-hydroxy-propane-1,1- diphosphonate disodique à partir de la solution aqueuse au moins saturée, sous la forme cristalline souhaitée comprenant de l'eau de cristallisation, en déclenchant la formation de cristaux à au moins 50°C, on isole le produit et sèche entre 15 et 60°C.

10. Procédé selon la revendication 9, caractérisé en ce qu'on choisit les conditions de réaction, de façon à ce que la neutralisation ait lieu entre 35 et 85°C, la formation de cristaux entre 50 et 70°C et la croissance cristalline entre 50 et 0°C.

11. Procédé selon la revendication 7, caractérisé en ce qu'on traite une forme cristalline du 3-amino-1-hydroxypropane-1,1-diphosphonate disodique d'une teneur en eau inférieure à 24% avec la quantité d'eau correspondante au moins nécessaire à la formation de la nouvelle forme cristalline comprenant de l'eau de cristallisation et avec la quantité d'eau correspondant au plus à l'inverse de la concentration de saturation moins 1 du 3-amino-1-hydroxypropane-1,1-diphosphonate disodique.

12. Procédé selon la revendication 11, caractérisé en ce qu'on agite avec de 2 à 3 fois la quantité d'eau.

13. Procédé selon l'une des revendications 7 à 12, caractérisé en ce qu'on réalise le séchage entre 18 et 25°C ou entre 35 et 40°C.

14. Procédé selon l'une des revendications 9 à 13, caractérisé en ce qu'on neutralise une suspension de 10 parties en poids d'acide 3-amino-1-hydroxypropane-1,1-diphosphonique dans 18 à 23 parties en volume d'eau entre 65 et 85°C avec la quantité de solution aqueuse de soude de 27,5 à 32,5% nécessaire pour former le 3-amino-1-hydroxypropane-1,1-diphosphonate disodique, on laisse refroidir la solution obtenue de 70 à 55°C, après déclenchement ou démarrage de la formation de cristaux on refroidit entre 20 et 25°C, on ajoute 7 à 12 parties en volume d'éthanol, on refroidit encore entre 5 et 0°C, on agite à cette température entre 1 et 15 heures, on isole le produit par aspiration sur filtre ou centrifugation, on lave par portions représentant en tout 7,5 à 12,5 parties en volume d'éthanol aqueux de 40 à 60%, ou on neutralise une suspension de 10 parties en poids d'acide 3-amino-1-hydroxypropane-1,1-diphosphonique dans 25 à 37,5 volumes d'eau à 35°C au début avec la quantité de solution aqueuse de soude de 27,5 à 32,5% nécessaire pour former le 3-amino-1-hydroxypropane-1,1-diphosphonate disodique, en réglant la vitesse d'addition de telle façon que la température monte entre 50 et 55°C, après déclenchement ou démarrage de la formation de cristaux on laisse refroidir entre 18 et 25°C, on isole le produit par aspiration sur filtre, on lave par portion avec en tout 15 à 25 parties en volume d'éthanol aqueux de 50 à 75%, et chaque fois on sèche jusqu'à poids constant entre 35 et 40°C.

15. Procédé selon l'une des revendications 7 à 14, caractérisé en ce qu'on sèche sous pression réduite.

16. Procédé selon l'une des revendications 7 à 15, caractérisé en ce qu'on sèche entre 5 et 20 mbar.

17. Procédé selon la revendication 7, caractérisé en ce qu'on fait agir sur une forme cristalline du 3-amino-1-hydroxypropane-1,1-diphosphonate disodique de teneur en eau inférieure à 24% en poids, entre 20 et 25°C de l'air humide, d'humidité relative comprise entre 90 et 99%, jusqu'à une augmentation de poids d'au moins 32,2% environ (compte tenu de la teneur en eau initiale), puis on abaisse l'humidité de l'air entre 35% environ et 80% environ.

18. Procédé selon les revendications 7, 11, 12 et 17, caractérisé en ce qu'on utilise comme forme solide de 3-amino-1-hydroxypropane-1,1- diphosphonate disodique de teneur en eau inférieure à 24% un 3-amino-1-hydroxypropane-1,1-diphosphonate disodique largement anhydre ou un 3-amino-1-hydroxypropane-1,1-diphosphonate d'une teneur en eau de 5 à 10% en poids, déterminée par chauffage à 180 °C.

19. Procédé selon l'une des revendications 7 à 18 pour préparer un 3-amino-1-hydroxypropane-1,1-diphosphonate disodique selon l'une des revendications 2, 3 et 5.

20. Utilisation de composé selon l'une des revendications 1 à 5 ou d'un composé qu'on peut obtenir selon l'une des revendications 7 à 19 pour produire des préparations pharmaceutiques destinées à l'administration entérale, adaptées au traitement entéral des troubles de l'échange calcium et/ou phosphate.

21. 3-amino-1-hydroxypropane-1,1-diphosphonate disodique selon la revendication 1, caractérisé par les paramètres de réseau suivants (valeurs d) et les intensités relatives de raies (intensités) de son diagramme de poudre de rayons X (chambre Guinier-de-Wolff, source de rayonnement: raie du cuivre $K_\alpha$):

| Valeurs d (Ångström) | Intensité |
|---|---|
| 10,1 | très faible |
| 9,1 | moyenne |
| 8,9 | faible |
| 6,6 | très faible |
| 5,91 | faible |
| 5,67 | très faible |
| 5,49 | forte |
| 4,90 | forte |
| 4,73 | moyenne |
| 4,54 | moyenne |
| 4,19 | faible |
| 3,61 | très faible |
| 3,13 | faible |
| 3,11 | faible |
| 3,06 | faible |
| 3,03 | très faible |
| 2,98 | faible |
| 2,91 | faible |
| 2,86 | moyenne |

22. Procédé de préparation d'un 3-amino-1-hydroxypropane-1,1-diphosphonate disodique selon la revendication 21, caractérisé en ce qu'on chauffe à 60 °C und suspension de 10 parties en poids d'acide 3-amino-1-hydroxypropa ne-1,1-diphosphonique dans 70 à 100 parties d'eau en poids environ, qu'on ajoute la quantité nécessaire de solution aqueuse de soude de concentration 27,5 à 32,5%, qu'on évapore à 75 °C, 50 parties d'eau en poids, qu'on ajoute 10 à 15 parties en volume d'alcool, qu'on refroidit à température ambiante, qu'on agite 1 heure entre 0 et 3 °C , on filtre par aspiration, lave avec de l'alcool à 60% environ et sèche à 120 °C jusqu'à poids constant.

23. 3-amino-1-hydroxypropane-1,1-diphosphonate disodique selon la revendication 1, caractérisé par les paramètres de réseau (valeur d) et les intensités relatives de raie (intensités) de son diagramme de poudre de rayon X (chambre Guinier-de-Wolff, source de rayonnement: raie du cuivre $K_\alpha$):

| Valeurs d (Ångström) | Intensité |
|---|---|
| 11,7 | forte |
| 7,8 | très forte |
| 6,8 | très forte |
| 5,83 | forte |
| 5,66 | forte |
| 5,38 | très faible |
| 4,76 | moyenne |
| 4,66 | moyenne |
| 4,29 | très faible |
| 4,20 | très faible |
| 4,13 | faible |
| 4,06 | moyenne |
| 3,99 | moyenne |
| 3,89 | moyenne |
| 3,82 | forte |
| 3,76 | très faible |
| 3,63 | moyenne |
| 3,51 | faible |
| 3,46 | forte |
| 3,27 | faible |
| 3,22 | très faible |
| 3,15 | forte |
| 3,08 | forte |
| 3,01 | faible |
| 2,94 | faible |
| 2,91 | forte |
| 2,89 | forte |
| 2,86 | faible |
| 2,82 | forte |
| 2,78 | moyenne |

24. Procédé de préparation d'un 3-amino-1-hydroxypropane-1,1-diphosphonate disodique selon la revendication 1, caractérisé en ce qu'on concentre fortement une solution aqueuse de 3-amino-1-hydroxypropane-1,1-diphosphonate à 75 °C, on fait cristalliser le produit par refroidissement lent dans l'intervalle de température de 45 à 0 °C, on filtre par aspiration et on sèche à température ambiante sous pression réduite jusqu'à poids constant.

**Revendications pour l'Etat contractant: AT**

1. Procédé de préparation de 3-amino-1-hydroxypropane-1,1-diphosphonate disodique cristallin, comprenant de l'eau de cristallisation de formule

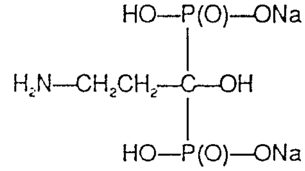

(I)

sous forme non-hygroscopique, caractérisé en ce qu'on fait cristalliser du 3-amino-1-hydroxypropane-1,1-diphosphonate disodique, à partir d'une solution aqueuse, en amorçant la cristallisation à au moins 50 °C, ou bien on traite une forme solide de 3-amino-1-hydroxypropane-1,1-diphosphonate disodique d'une teneur en eau inférieure à 24% avec de l'eau, on isole chaque fois le produit et on sèche entre 15 et 60 °C.

2. Procédé selon la revendication 1, caractérisé en ce qu'on concentre une solution aqueuse de 10 à 55% de 3-amino-1-hydroxypropane-1,1-diphosphonate disodique entre 70 et 80 °C jusqu'à concentration de saturation du 3-amino-1-hydroxypropane-1,1-diphosphonate disodique et on provoque la cristallisation par refroidissement entre 0 et 5 °C, ou on refroidit une solution aqueuse de 35 à 45% de 3-amino-1-hydroxypropane-1,1-diphosphonate disodique, entre 70 et 80 °C au départ, jusqu'à une température qui n'est pas inférieure à 55 °C pour commencer la cristallisation, et on provoque la cristallisation par refroidissement entre 20 et 25 °C, et addition de 35 à 45% en volume d'éthanol (par rapport à la quantité totale d'eau) et on refroidit entre 0 et 5 °C.

3. Procédé de préparation de 3-amino-1-hydroxypropane-1,1-diphosphonate disodique cristallin comprenant de l'eau de cristallisation par réaction d'acide 3-amino-1-hydroxypropane-1,1-diphosphonique avec un sel basique de sodium approprié pour sa neutralisation partielle, caractérisé en ce que pour obtenir un produit non hygroscopique d'acide 3-amino-1-hydroxypropane-1,1-diphosphonique en suspension aqueuse et/ou solution on fait réagir avec la quantité nécessaire de solution aqueuse de soude, on fait cristalliser le 3-amino-1-hydroxypropane-1,1- diphosphonate disodique à partir de la solution aqueuse au moins saturée, sous la forme cristalline souhaitée comprenant de l'eau de cristallisation, on isole le produit et sèche entre 15 et 60 °C.

4. Procédé selon la revendication 3, caractérisé en ce qu'on choisit les conditions de réaction, de façon à ce que la neutralisation ait lieur entre 35 et 85 °C, la formation de cristaux entre 50 et 75 °C et la croissance cristalline entre 50 °C et 0 °C.

5. Procédé selon la revendication 1, caractérisé en ce qu'on traite une forme solide du 3-amino-1-hydroxypropane-1,1-diphosphonate disodique d'une teneur en eau inférieure à 24% avec la quantité d'eau correspondante au moins nécessaire à la formation de la nouvelle forme cristalline comprenant de l'eau de cristallisation et avec la quantité d'eau correspondant au plus à l'inverse de la concentration de saturation moins 1 du 3-amino-1-hydroxypropane-1,1-diphosphonate disodique.

6. Procédé selon la revendication 5, caractérisé en ce qu'on agite avec de 2 à 3 fois la quantité d'eau.

7. Procédé selon l'une des revendications 1 à 7, caractérisé en ce qu'on réalis le séchage entre 18 et 25 °C ou entre 35 et 40 °C.

8. Procédé selon les revendications 3, 4 ou 7 caractérisé en ce qu'on neutralise une suspension de 10 parties en poids d'acide 3-amino-1-hydroxypropane-1,1-diphosphonique dans 18 à 23 parties en volume d'eau entre 65 et 85 °C avec la quantité de solution aqueuse de soude de 27,5 à 32,5% nécessaire pour former le 3-amino-1-hydroxypropane-1,1-diphosphonate disodique, on laisse refroidir la solution obtenue de 70 à 55 °C, après déclenchement ou démarrage de la formation de cristaux on refroidit entre 20 et 25 °C, on ajoute 7 à 12 parties en volume d'éthanol, on refroidit encore entre 5 et 0 °C, on agite à cette température entre 1 et 15 heures, on isole le produit par aspiration sur filtre ou centrifugation, on lave par portions représentant en tout 7,5 à 12,5 parties en volume d'éthanol aqueux de 40 à 60%, ou on neutralise une suspension de 10 parties en poids d'acide 3-amino-1-hydroxypropane-1,1-diphosphonique dans 25 à 37,5 volumes d'eau à 35 °C au début avec la quantité de solution aqueuse de soude de 27,5 à 32,5% nécessaire pour former le 3-amino-1-hydroxypropane-1,1-diphosphonate disodique, en réglant la vitesse d'addition de telle façon que la température monte entre 50 et 55 °C, après déclenchement ou démarrage de la formation de cristaux on laisse refroidir entre 18 et 25 °C, on isole le produit par aspiration sur filtre, on lave par portion avec en tout 15 à 25 parties en volume d'éthanol aqueux de 50 à 75%, et chaque fois on sèche jusqu'à poids constant entre 35 et 40 °C.

9. Procédé selon l'une des revendications 1 à 8, caractérisé en ce qu'on sèche sous pression réduite.

10. Procédé selon l'une des revendications 1 à 9, caractérisé en ce qu'on sèche entre 5 et 20 mbar.

11. Procédé selon la revendication 1, caractérisé en ce qu'on fait agir sur une forme cristalline du 3-amino-1-hydroxypropane-1,1-diphosphonate disodique de teneur en eau inférieure à 24% en poids, entre 20 et 25 °C de l'air humide, d'humidité relative comprise entre 90 et 99%, jusqu'à une augmentation de poids d'au moins 32,2% environ (compte tenu de la teneur en eau initiale), puis on abaisse l'humidité de l'air entre 35% environ et 80% environ.

12. Procédé selon l'une des revendications 1, 5, 6 et 11 caractérisé en ce qu'on utilise comme forme solide de 3-amino-1-hydroxypropane-1,1-diphosphonate disodique de teneur en eau inférieure à 24% un 3-amino-1-hydroxypropane-1,1-diphosphonate disodique largement anhydre ou un 3-amino-1-hydroxypropane-1,1- diphosphonate d'une teneur en eau de 5 à 10% en poids, déterminée par chauffage à 180 °C.

13. Procédé selon l'une des revendications 1 à 12, caractérisé en ce qu'on prépare du 3-amino-1-hydroxypropane-1,1-diphosphonate disodique cristallin, comprenant de l'eau de cristallisation sous forme non hygroscopique avec une teneur en eau de 24,1 à 24,5% en poids.

14. Procédé selon l'une des revendications 1 à 12, caractérisé en ce qu'on prépare du 3-amino-1-hydroxypropane-1,1-diphosphonate disodique

sous forme de pentahydrate non hygroscopique.

15. Procédé selon l'une des revendications 1 à 12, caractérisé en ce qu'on prépare du 3-amino-1-hydroxypropane-1,1-diphosphonate disodique cristallin, avec de l'eau de cristallisation sous forme non hygroscopique, caractérisé par les paramètres de réseau suivants (valeurs d) et par les intensités relatives de raies (intensités) de son diagramme de poudre de rayons X (chambre Guinier-de-Wolff, source de rayonnement raie du cuivre $K_\alpha$):

| Valeurs d (Ångström) | Intensité |
|---|---|
| 10,2 | moyenne |
| 9,9 | moyenne |
| 9,2 | très forte |
| 5,91 | très forte |
| 5,57 | forte |
| 5,42 | très faible |
| 5,30 | forte |
| 5,14 | moyenne |
| 5,02 | très forte |
| 4,97 | très forte |
| 4,63 | très faible |
| 4,41 | forte |
| 4,16 | forte |
| 4,07 | faible |
| 4,04 | moyenne |
| 3,95 | très faible |
| 3,75 | forte |
| 3,67 | faible |
| 3,63 | moyenne |
| 3,61 | très faible |
| 3,58 | très faible |
| 3,51 | moyenne |
| 3,43 | forte |
| 3,38 | moyenne |
| 3,15 | moyenne |
| 3,14 | moyenne |
| 3,09 | moyenne |
| 3,03 | très forte |
| 3,01 | très forte |
| 2,98 | forte |
| 2,97 | très faible |
| 2,91 | très forte |
| 2,82 | forte |
| 2,80 | moyenne |
| 2,78 | moyenne |
| 2,75 | forte |
| 2,73 | faible |
| 2,71 | faible |
| 2,69 | faible |
| 2,67 | moyenne |
| 2,66 | moyenne |
| 2,63 | forte |
| 2,62 | forte |
| 2,61 | forte |
| 2,58 | très forte |
| 2,57 | très forte |

16. Procédé de production de préparations pharmaceutiques destinées à l'administration entérale à des êtres à sang chaud, comprenant du 3-amino-1-hydroxypropane-1,1-diphosphonate disodique, caractérisé en ce qu'on mélange avec les additifs pharmaceutiques usuels du 3-amino-1-hydroxypropane-1,1-diphosphonate disodique sous forme cristalline, comprenant de l'eau de cristallisation, non hygroscopique, obtenu selon l'une des revendications 1 à 16.

17. Procédé de préparation de 3-amino-1-hydroxypropane-1,1-diphosphonate disodique, cristallin, comprenant de l'eau de cristallisation, caractérisé par les paramètres de réseau suivants (valeurs d) et les intensités relatives de raies (intensités) de son diagramme de poudre de rayons X (chambre Guinier-de-Wolff, source de rayonnement: raie du cuivre $K_\alpha$):

| Valeurs d (Ångström) | Intensité |
|---|---|
| 10,1 | très faible |
| 9,1 | moyenne |
| 8,9 | faible |
| 6,6 | très faible |
| 5,91 | faible |
| 5,67 | très faible |
| 5,49 | forte |
| 4,90 | forte |
| 4,73 | moyenne |
| 4,54 | moyenne |
| 4,19 | faible |
| 3,61 | très faible |
| 3,13 | faible |
| 3,11 | faible |
| 3,06 | faible |
| 3,03 | très faible |
| 2,98 | faible |
| 2,91 | faible |
| 2,86 | moyenne |

caractérisé en ce qu'on chauffe à 60 °C une suspension de 10 parties en poids d'acide 3-amino-1-hydroxypropane-1,1-diphosphonique dans 70 à 100 parties d'eau en poids environ, qu'on ajoute la quantité nécessaire de solution aqueuse de soude de concentration 27,5 à 32,5%, qu'on évapore à 75 °C 50 parties d'eau en poids, qu'on ajoute 10 à 15 parties en volume d'alcohol, qu'on refroidit à température ambiante, qu'on agite 1 heure entre 0 et 3 °C, on filtre par aspiration, lave avec de l'alcool à 60% environ et sèche à 120 °C jusqu'à poids constant.

18. Procédé de préparation de 3-amino-1-hydroxypropane-1,1-diphosphonate disodique cristallin à eau de cristallisation caractérisé par les paramètres de réseau (valeur d) et les intensités relatives de raie (intensités) de son diagramme de poudre de rayon X (chambre Guinier-de Wolff, source de rayonnement: raie du cuivre $K_\alpha$):

| Valeurs d (Ångström) | Intensité |
|---|---|
| 11,7 | forte |
| 7,8 | très forte |
| 6,8 | très forte |
| 5,83 | forte |
| 5,66 | forte |
| 5,38 | très faible |
| 4,76 | moyenne |
| 4,66 | moyenne |
| 4,29 | très faible |
| 4,20 | très faible |
| 4,13 | faible |
| 4,06 | moyenne |
| 3,99 | moyenne |
| 3,89 | moyenne |
| 3,82 | forte |
| 3,76 | très faible |
| 3,63 | moyenne |
| 3,51 | faible |
| 3,46 | forte |
| 3,27 | faible |
| 3,22 | très faible |
| 3,15 | forte |
| 3,08 | forte |
| 3,01 | faible |
| 2,94 | faible |
| 2,91 | forte |
| 2,89 | forte |
| 2,86 | faible |
| 2,82 | forte |
| 2,78 | moyenne |

caractérisé en ce qu'on concentre fortement une solution aqueuse de 3-amino-1-hydroxypropane-1,1-diphosphonate à 75°C, on fait cristalliser le produit par refroidissement lent dans l'intervalle de température de 45 à 0°C, on filtre par aspiration et on sèche à température ambiante sous pression réduite jusqu'à poids constant.